# EUROPEAN PATENT APPLICATION

(11) **EP 2 100 620 A1**
(43) Date of publication of application: **16.09.2009**
(21) Application number: 09008339.5
(22) Date of filing: 18.09.2000
(51) Int. Cl.: A61K 48/00, C12N 15/00, C12N 15/88, C07H 21/04

(54) **Nucleic acids encoding polyepitope polypeptides**

(30) Priority: 16.09.1999 US 398534; 16.09.1999 US 154665 P; 09.12.1999 US 458173; 09.12.1999 US 169846 P
(62) Divisional of application: 00965119.1
(71) Applicant: Eisai Corporation of North America, Woodcliff Lake, NJ 07677 (US)
(72) Inventor: The designation of the inventor has not yet been filed
(74) Representative: Dzieglewska, Hanna Eva

(57) **Abstract**

The invention provides nucleic acids encoding polyepitope polypeptides containing multiple epitopes from one or more proteins. The polyepitope polypeptides are useful as treatments for pathogenic agents or tumors.

SEQUENCE LISTING

<110> Eisai Corporation of North America

<120> NUCLEIC ACIDS ENCODING POLYEPITOPE POLYPEPTIDES

<130> 27.20.77866/01

<140> PCT/US00/25559
<141> 2000-09-18

<150> US 09/398,534
<151> 1999-09-16

<150> US 60/154,665
<151> 1999-09-16

<150> US 09/458,173
<151> 1999-12-09

<150> US 60/169,846
<151> 1999-12-09

<160> 163

<170> FastSEQ for Windows Version 4.0

<210> 1
<211> 9
<212> PRT
<213> Homo sapiens

<400> 1
Ala Ala Arg Ala Val Phe Leu Ala Leu
 1               5

<210> 2
<211> 8
<212> PRT
<213> Homo sapiens

<400> 2
Tyr Arg Pro Arg Pro Arg Arg Tyr
 1               5

<210> 3
<211> 9
<212> PRT
<213> Homo sapiens

<400> 3
Glu Ala Asp Pro Thr Gly His Ser Tyr
 1               5

<210> 4
<211> 9
<212> PRT
<213> Homo sapiens

<400> 4
Ser Ala Tyr Gly Glu Pro Arg Lys Leu
 1               5

<210> 5
<211> 9
<212> PRT
<213> Homo sapiens

<400> 5
Glu Val Asp Pro Ile Gly His Leu Tyr
1               5

<210> 6
<211> 9
<212> PRT
<213> Homo sapiens

<400> 6
Phe Leu Trp Gly Pro Arg Ala Leu Val
1               5

<210> 7
<211> 7
<212> PRT
<213> Homo sapiens

<400> 7
Gly Ile Gly Ile Leu Thr Val
1               5

<210> 8
<211> 8
<212> PRT
<213> Homo sapiens

<400> 8
Ile Leu Thr Val Ile Leu Gly Val
1               5

<210> 9
<211> 9
<212> PRT
<213> Homo sapiens

<400> 9
Ser Thr Ala Pro Pro Ala His Gly Val
1               5

<210> 10
<211> 9
<212> PRT
<213> Homo sapiens

<400> 10
Glu Glu Lys Leu Ile Val Val Leu Phe
1               5

<210> 11
<211> 9
<212> PRT
<213> Homo sapiens

<400> 11
Met Leu Leu Ala Val Leu Tyr Cys Leu
1               5

<210> 12
<211> 9
<212> PRT
<213> Homo sapiens

<400> 12
Ser Glu Ile Trp Arg Asp Ile Asp Phe
1               5

<210> 13

<211> 9
<212> PRT
<213> Homo sapiens

<400> 13
Ala Phe Leu Pro Trp His Arg Leu Phe
1               5

<210> 14
<211> 9
<212> PRT
<213> Homo sapiens

<400> 14
Tyr Met Asn Gly Thr Met Ser Gln Val
1               5

<210> 15
<211> 9
<212> PRT
<213> Homo sapiens

<400> 15
Lys Thr Trp Gly Gln Tyr Trp Gln Val
1               5

<210> 16
<211> 9
<212> PRT
<213> Homo sapiens

<400> 16
Ile Thr Asp Gln Val Pro Phe Ser Val
1               5

<210> 17
<211> 9
<212> PRT
<213> Homo sapiens

<400> 17
Tyr Leu Glu Pro Gly Pro Thr Val Ala
1               5

<210> 18
<211> 10
<212> PRT
<213> Homo sapiens

<400> 18
Leu Leu Asp Gly Thr Ala Thr Leu Arg Leu
1               5                   10

<210> 19
<211> 10
<212> PRT
<213> Homo sapiens

<400> 19
Glu Leu Asn Glu Ala Leu Glu Leu Glu Lys
1               5                   10

<210> 20
<211> 9
<212> PRT
<213> Homo sapiens

<400> 20

Ser Thr Pro Pro Pro Gly Thr Arg Val
1               5

<210> 21
<211> 11
<212> PRT
<213> Homo sapiens

<400> 21
Leu Leu Pro Glu Asn Asn Val Leu Ser Pro Leu
1               5                   10

<210> 22
<211> 9
<212> PRT
<213> Homo sapiens

<400> 22
Leu Leu Gly Arg Asn Ser Phe Glu Val
1               5

<210> 23
<211> 9
<212> PRT
<213> Homo sapiens

<400> 23
Arg Met Pro Glu Ala Ala Pro Pro Val
1               5

<210> 24
<211> 9
<212> PRT
<213> Homo sapiens

<400> 24
Lys Ile Phe Gly Ser Leu Ala Phe Leu
1               5

<210> 25
<211> 9
<212> PRT
<213> Homo sapiens

<400> 25
Ile Ile Ser Ala Val Val Gly Ile Leu
1               5

<210> 26
<211> 9
<212> PRT
<213> Homo sapiens

<400> 26
Cys Leu Thr Ser Thr Val Gln Leu Val
1               5

<210> 27
<211> 8
<212> PRT
<213> Homo sapiens

<400> 27
Tyr Leu Glu Asp Val Arg Leu Val
1               5

<210> 28
<211> 9

<212> PRT
<213> Homo sapiens

<400> 28
Val Leu Val Lys Ser Pro His Asn Val
1               5

<210> 29
<211> 12
<212> PRT
<213> Homo sapiens

<400> 29
Arg Phe Arg Glu Leu Val Ser Glu Phe Ser Arg Met
1               5                   10

<210> 30
<211> 10
<212> PRT
<213> Homo sapiens

<400> 30
Leu Leu Arg Leu Ser Glu Pro Ala Glu Leu
1               5                   10

<210> 31
<211> 9
<212> PRT
<213> Homo sapiens

<400> 31
Asp Leu Pro Thr Gln Glu Pro Ala Leu
1               5

<210> 32
<211> 6
<212> PRT
<213> Homo sapiens

<400> 32
Lys Leu Gln Cys Val Asp
1               5

<210> 33
<211> 10
<212> PRT
<213> Homo sapiens

<400> 33
Val Leu Val Ala Ser Arg Gly Arg Ala Val
1               5                   10

<210> 34
<211> 9
<212> PRT
<213> Homo sapiens

<400> 34
Val Leu Val His Pro Gln Trp Val Leu
1               5

<210> 35
<211> 10
<212> PRT
<213> Homo sapiens

<400> 35
Asp Met Ser Leu Leu Lys Asn Arg Phe Leu

1                   5                        10

<210> 36
<211> 9
<212> PRT
<213> Hepatitis B virus

<400> 36
Gln Trp Asn Ser Thr Ala Phe His Gln
1                   5

<210> 37
<211> 7
<212> PRT
<213> Hepatitis B virus

<400> 37
Val Leu Gln Ala Gly Phe Phe
1                   5

<210> 38
<211> 8
<212> PRT
<213> Hepatitis B virus

<400> 38
Leu Leu Leu Cys Leu Ile Phe Leu
1                   5

<210> 39
<211> 8
<212> PRT
<213> Hepatitis B virus

<400> 39
Leu Leu Asp Tyr Gln Gly Met Leu
1                   5

<210> 40
<211> 6
<212> PRT
<213> Hepatitis B virus

<400> 40
Leu Leu Val Pro Phe Val
1                   5

<210> 41
<211> 10
<212> PRT
<213> Hepatitis B virus

<400> 41
Ser Ile Leu Ser Pro Phe Met Pro Leu Leu
1                   5                        10

<210> 42
<211> 9
<212> PRT
<213> Hepatitis B virus

<400> 42
Pro Leu Leu Pro Ile Phe Phe Cys Leu
1                   5

<210> 43
<211> 10
<212> PRT

<213> Hepatitis B virus

<400> 43
Ile Leu Ser Thr Leu Pro Glu Thr Thr Val
1               5                   10

<210> 44
<211> 10
<212> PRT
<213> Hepatitis B virus

<400> 44
Phe Leu Pro Ser Asp Phe Phe Pro Ser Val
1               5                   10

<210> 45
<211> 9
<212> PRT
<213> Hepatitis B virus

<400> 45
Lys Leu His Leu Tyr Ser His Pro Ile
1               5

<210> 46
<211> 9
<212> PRT
<213> Hepatitis B virus

<400> 46
Ala Leu Met Pro Leu Tyr Ala Cys Ile
1               5

<210> 47
<211> 9
<212> PRT
<213> Hepatitis B virus

<400> 47
His Leu Tyr Ser His Pro Ile Ile Leu
1               5

<210> 48
<211> 9
<212> PRT
<213> Hepatitis B virus

<400> 48
Phe Leu Leu Ser Leu Gly Ile His Leu
1               5

<210> 49
<211> 9
<212> PRT
<213> Hepatitis B virus

<400> 49
His Leu Leu Val Gly Ser Ser Gly Leu
1               5

<210> 50
<211> 9
<212> PRT
<213> Hepatitis B virus

<400> 50
Gly Leu Ser Arg Tyr Val Ala Arg Leu
1               5

<210> 51
<211> 9
<212> PRT
<213> Hepatitis B virus

<400> 51
Leu Leu Ala Gln Phe Thr Ser Ala Ile
1               5

<210> 52
<211> 9
<212> PRT
<213> Hepatitis B virus

<400> 52
Tyr Met Asp Asp Val Val Leu Gly Ala
1               5

<210> 53
<211> 9
<212> PRT
<213> Hepatitis B virus

<400> 53
Gly Leu Tyr Ser Ser Thr Val Pro Val
1               5

<210> 54
<211> 5
<212> PRT
<213> Hepatitis B virus

<400> 54
Asn Leu Ser Trp Leu
1               5

<210> 55
<211> 9
<212> PRT
<213> Human Papilloma virus

<400> 55
Lys Leu Pro Gln Leu Cys Thr Glu Leu
1               5

<210> 56
<211> 9
<212> PRT
<213> Human Papilloma virus

<400> 56
Leu Gln Thr Thr Ile His Asp Ile Ile
1               5

<210> 57
<211> 9
<212> PRT
<213> Human Papilloma virus

<400> 57
Phe Ala Phe Arg Asp Leu Cys Ile Val
1               5

<210> 58
<211> 9
<212> PRT
<213> Human Papilloma virus

<400> 58
Tyr Met Leu Asp Leu Gln Pro Glu Thr
1               5

<210> 59
<211> 9
<212> PRT
<213> Human Papilloma virus

<400> 59
Thr Leu His Glu Tyr Met Leu Asp Leu
1               5

<210> 60
<211> 9
<212> PRT
<213> Human Papilloma virus

<400> 60
Leu Leu Met Gly Thr Leu Gly Ile Val
1               5

<210> 61
<211> 8
<212> PRT
<213> Human Papilloma virus

<400> 61
Thr Leu Gly Ile Val Cys Pro Ile
1               5

<210> 62
<211> 12
<212> PRT
<213> Human Papilloma virus

<400> 62
Leu Leu Met Gly Thr Leu Gly Ile Val Cys Pro Ile
1               5                   10

<210> 63
<211> 25
<212> PRT
<213> Homo sapiens

<400> 63
Met Ala Ile Ser Gly Val Pro Val Leu Gly Phe Phe Ile Ile Ala Val
1               5                   10                  15
Leu Met Ser Ala Gln Glu Ser Trp Ala
            20                  25

<210> 64
<211> 20
<212> PRT
<213> Human Papilloma virus

<400> 64
Ala Met Phe Gln Asp Pro Gln Glu Arg Pro Arg Lys Leu Pro Gln Leu
1               5                   10                  15
Cys Thr Glu Leu
            20

<210> 65
<211> 24
<212> PRT
<213> Human Papilloma virus

<400> 65
Leu Leu Arg Arg Glu Val Tyr Asp Phe Ala Phe Arg Asp Leu Cys Ile
1               5                   10                  15
Val Tyr Arg Asp Gly Asn Pro Tyr
            20

<210> 66
<211> 23
<212> PRT
<213> Human Papilloma virus

<400> 66
Lys Ile Ser Glu Tyr Arg His Tyr Cys Tyr Ser Leu Tyr Gly Thr Thr
1               5                   10                  15
Leu Glu Gln Gln Tyr Asn Lys
            20

<210> 67
<211> 19
<212> PRT
<213> Human Papilloma virus

<400> 67
Thr Leu His Glu Tyr Met Leu Asp Leu Gln Pro Glu Thr Thr Asp Leu
1               5                   10                  15
Tyr Ser Tyr

<210> 68
<211> 14
<212> PRT
<213> Human Papilloma virus

<400> 68
Gln Ala Glu Pro Asp Arg Ala His Tyr Asn Ile Val Thr Phe
1               5                   10

<210> 69
<211> 17
<212> PRT
<213> Human Papilloma virus

<400> 69
Leu Leu Met Gly Thr Leu Gly Ile Val Cys Pro Ile Cys Ser Gln Lys
1               5                   10                  15
Pro

<210> 70
<211> 9
<212> PRT
<213> Human Papilloma virus

<400> 70
Ile Ser Glu Tyr Arg His Tyr Cys Tyr
1               5

<210> 71
<211> 9
<212> PRT
<213> Human Papilloma virus

<400> 71
Phe Gln Asp Pro Gln Glu Arg Pro Arg
1               5

<210> 72
<211> 9

<212> PRT
<213> Human Papilloma virus

<400> 72
Arg Arg Glu Val Tyr Asp Phe Ala Phe
1               5

<210> 73
<211> 9
<212> PRT
<213> Human Papilloma virus

<400> 73
Thr Thr Leu Glu Gln Gln Tyr Asn Lys
1               5

<210> 74
<211> 10
<212> PRT
<213> Human papilloma virus

<400> 74
Phe Gln Asp Pro Gln Glu Arg Pro Arg Lys
1               5                   10

<210> 75
<211> 10
<212> PRT
<213> Human Papilloma virus

<400> 75
Ile Ser Glu Tyr Arg His Tyr Cys Tyr Ser
1               5                   10

<210> 76
<211> 10
<212> PRT
<213> Human Papilloma virus

<400> 76
Lys Ile Ser Glu Tyr Arg His Tyr Cys Tyr
1               5                   10

<210> 77
<211> 10
<212> PRT
<213> Human Papilloma virus

<400> 77
Gly Thr Thr Leu Glu Gln Gln Tyr Asn Lys
1               5                   10

<210> 78
<211> 9
<212> PRT
<213> Human Papilloma virus

<400> 78
Lys Ile Ser Glu Tyr Arg His Tyr Cys
1               5

<210> 79
<211> 10
<212> PRT
<213> Human Papilloma virus

<400> 79
Tyr Cys Tyr Ser Ile Tyr Gly Thr Thr Leu

1               5                   10

<210> 80
<211> 10
<212> PRT
<213> Human Papilloma virus

<400> 80
Ser Glu Tyr Arg His Tyr Cys Tyr Ser Leu
1               5                   10

<210> 81
<211> 9
<212> PRT
<213> Human Papilloma virus

<400> 81
Ala Met Phe Gln Asp Pro Gln Glu Arg
1               5

<210> 82
<211> 9
<212> PRT
<213> Human Papilloma virus

<400> 82
Leu Leu Arg Arg Glu Val Tyr Asp Phe
1               5

<210> 83
<211> 9
<212> PRT
<213> Human Papilloma virus

<400> 83
Ile Val Tyr Arg Asp Gly Asn Pro Tyr
1               5

<210> 84
<211> 9
<212> PRT
<213> Human Papilloma virus

<400> 84
Val Tyr Asp Phe Ala Phe Arg Asp Leu
1               5

<210> 85
<211> 9
<212> PRT
<213> Human Papilloma virus

<400> 85
Cys Tyr Ser Leu Tyr Gly Thr Thr Leu
1               5

<210> 86
<211> 9
<212> PRT
<213> Human Papilloma virus

<400> 86
Glu Tyr Arg His Tyr Cys Tyr Ser Leu
1               5

<210> 87
<211> 9
<212> PRT

<213> Human Papilloma virus

<400> 87
Asp Pro Gln Glu Arg Pro Arg Lys Leu
1               5

<210> 88
<211> 9
<212> PRT
<213> Human Papilloma virus

<400> 88
His Tyr Cys Tyr Ser Leu Tyr Gly Thr
1               5

<210> 89
<211> 9
<212> PRT
<213> Human Papilloma virus

<400> 89
Asp Phe Ala Phe Arg Asp Leu Cys Ile
1               5

<210> 90
<211> 10
<212> PRT
<213> Human Papilloma virus

<400> 90
Leu Tyr Gly Thr Thr Leu Glu Gln Gln Tyr
1               5                   10

<210> 91
<211> 10
<212> PRT
<213> Human Papilloma virus

<400> 91
His Tyr Cys Tyr Ser Leu Tyr Gly Thr Thr
1               5                   10

<210> 92
<211> 10
<212> PRT
<213> Human Papilloma virus

<400> 92
Glu Val Tyr Asp Phe Ala Phe Arg Asp Leu
1               5                   10

<210> 93
<211> 10
<212> PRT
<213> Human Papilloma virus

<400> 93
Glu Tyr Arg His Tyr Cys Tyr Ser Leu Tyr
1               5                   10

<210> 94
<211> 10
<212> PRT
<213> Human Papilloma virus

<400> 94
Val Tyr Asp Phe Ala Phe Arg Asp Leu Cys
1               5                   10

<210> 95
<211> 9
<212> PRT
<213> Human Papilloma virus

<400> 95
Gln Ala Glu Pro Asp Arg Ala His Tyr
1               5

<210> 96
<211> 9
<212> PRT
<213> Human Papilloma virus

<400> 96
Ile Val Cys Pro Ile Cys Ser Gln Lys
1               5

<210> 97
<211> 8
<212> PRT
<213> Human Papilloma virus

<400> 97
Gln Pro Glu Thr Thr Asp Leu Tyr
1               5

<210> 98
<211> 10
<212> PRT
<213> Human Papilloma virus

<400> 98
Gln Ala Glu Pro Asp Arg Ala His Tyr Asn
1               5                   10

<210> 99
<211> 10
<212> PRT
<213> Human Papilloma virus

<400> 99
Asp Leu Gln Pro Glu Thr Thr Asp Leu Tyr
1               5                   10

<210> 100
<211> 9
<212> PRT
<213> Human Papilloma virus

<400> 100
Leu Met Gly Thr Leu Gly Ile Val Cys
1               5

<210> 101
<211> 9
<212> PRT
<213> Human Papilloma virus

<400> 101
Met Leu Asp Leu Gln Pro Glu Thr Thr
1               5

<210> 102
<211> 9
<212> PRT
<213> Human Papilloma virus

<400> 102
Thr Leu Gly Ile Val Cys Pro Ile Cys
1               5

<210> 103
<211> 9
<212> PRT
<213> Human Papilloma virus

<400> 103
Asp Leu Gln Pro Glu Thr Thr Asp Leu
1               5

<210> 104
<211> 9
<212> PRT
<213> Human Papilloma virus

<400> 104
Gly Thr Leu Gly Ile Val Cys Pro Ile
1               5

<210> 105
<211> 10
<212> PRT
<213> Human Papilloma virus

<400> 105
Tyr Met Leu Asp Leu Gln Pro Glu Thr Thr
1               5                   10

<210> 106
<211> 9
<212> PRT
<213> Human Papilloma virus

<400> 106
Leu Gln Pro Glu Thr Thr Asp Leu Tyr
1               5

<210> 107
<211> 10
<212> PRT
<213> Human Papilloma virus

<400> 107
Leu Leu Met Gly Thr Leu Gly Ile Val Cys
1               5                   10

<210> 108
<211> 9
<212> PRT
<213> Human Papilloma virus

<400> 108
Thr Pro Thr Leu His Glu Tyr Met Leu
1               5

<210> 109
<211> 9
<212> PRT
<213> Human Papilloma virus

<400> 109
Arg Ala His Tyr Asn Ile Val Thr Phe
1               5

<210> 110
<211> 9
<212> PRT
<213> Human Papilloma virus

<400> 110
Glu Pro Asp Arg Ala His Tyr Asn Ile
1               5

<210> 111
<211> 4
<212> PRT
<213> Homo sapiens

<400> 111
Lys Asp Glu Leu
1

<210> 112
<211> 5
<212> PRT
<213> Homo sapiens

<400> 112
Lys Phe Glu Arg Gln
1               5

<210> 113
<211> 5
<212> PRT
<213> Homo sapiens

<400> 113
Gln Arg Glu Phe Lys
1               5

<210> 114
<211> 17
<212> PRT
<213> Homo sapiens

<400> 114
Ala Val Lys Arg Pro Ala Ala Thr Lys Lys Ala Gly Gln Ala Lys Lys
1               5                   10                  15
Lys

<210> 115
<211> 17
<212> PRT
<213> Homo sapiens

<400> 115
Arg Pro Ala Ala Thr Lys Lys Ala Gly Gln Ala Lys Lys Lys Lys Leu
1               5                   10                  15
Asp

<210> 116
<211> 19
<212> PRT
<213> Homo sapiens

<400> 116
Ala Val Lys Arg Pro Ala Ala Thr Lys Lys Ala Gly Gln Ala Lys Lys
1               5                   10                  15
Lys Leu Asp

<210> 117
<211> 9
<212> PRT
<213> Homo sapiens

<400> 117
Tyr Leu Glu Pro Ala Ile Ala Lys Tyr
1               5

<210> 118
<211> 10
<212> PRT
<213> Homo sapiens

<400> 118
Phe Leu Pro Ser Asp Tyr Phe Pro Ser Val
1               5                   10

<210> 119
<211> 10
<212> PRT
<213> Homo sapiens

<400> 119
Lys Val Phe Pro Tyr Ala Leu Ile Asn Lys
1               5                   10

<210> 120
<211> 9
<212> PRT
<213> Homo sapiens

<400> 120
Ala Val Asp Leu Tyr His Phe Leu Lys
1               5

<210> 121
<211> 9
<212> PRT
<213> Homo sapiens

<400> 121
Ala Tyr Ile Asp Asn Tyr Asn Lys Phe
1               5

<210> 122
<211> 9
<212> PRT
<213> Human Papilloma virus

<400> 122
His Thr Met Leu Cys Met Cys Cys Lys
1               5

<210> 123
<211> 9
<212> PRT
<213> Human Papilloma virus

<400> 123
Gln Leu Phe Leu Asn Thr Leu Ser Phe
1               5

<210> 124
<211> 9
<212> PRT
<213> Human Papilloma virus

<400> 124
Lys Leu Pro Asp Leu Cys Thr Glu Leu
1               5

<210> 125
<211> 10
<212> PRT
<213> Human Papilloma virus

<400> 125
Gly Leu Tyr Asn Leu Leu Ile Arg Cys Leu
1               5                   10

<210> 126
<211> 117
<212> PRT
<213> Artificial Sequence

<220>
<223> Artificial fusion sequence

<400> 126
Ala Met Phe Gln Asp Pro Gln Glu Arg Pro Arg Lys Leu Pro Gln Leu
1               5                   10                  15
Cys Thr Glu Leu Leu Leu Arg Arg Glu Val Tyr Asp Phe Ala Phe Arg
            20                  25                  30
Asp Leu Cys Ile Val Tyr Arg Asp Gly Asn Pro Tyr Lys Ile Ser Glu
        35                  40                  45
Tyr Arg His Tyr Cys Tyr Ser Leu Tyr Gly Thr Thr Leu Glu Gln Gln
    50                  55                  60
Tyr Asn Lys Thr Leu His Glu Tyr Met Leu Asp Leu Gln Pro Glu Thr
65                  70                  75                  80
Thr Asp Leu Tyr Ser Tyr Gln Ala Glu Pro Asp Arg Ala His Tyr Asn
                85                  90                  95
Ile Val Thr Phe Leu Leu Met Gly Thr Leu Gly Ile Val Cys Pro Ile
            100                 105                 110
Cys Ser Gln Lys Pro
        115

<210> 127
<211> 10
<212> PRT
<213> Human Papilloma virus

<400> 127
Leu Thr Glu Val Phe Glu Phe Ala Phe Lys
1               5                   10

<210> 128
<211> 9
<212> PRT
<213> Human Papilloma virus

<400> 128
Gly Leu Tyr Asn Leu Leu Ile Arg Cys
1               5

<210> 129
<211> 9
<212> PRT
<213> Human Papilloma virus

<400> 129
Ser Leu Gln Asp Ile Glu Ile Thr Cys
1               5

<210> 130

<211> 10
<212> PRT
<213> Human Papilloma virus

<400> 130
Ser Leu Gln Asp Ile Glu Ile Thr Cys Val
1               5                   10

<210> 131
<211> 9
<212> PRT
<213> Human Papilloma virus

<400> 131
Leu Gln Asp Ile Glu Ile Thr Cys Val
1               5

<210> 132
<211> 9
<212> PRT
<213> Human Papilloma virus

<400> 132
Lys Thr Val Leu Glu Leu Thr Glu Val
1               5

<210> 133
<211> 9
<212> PRT
<213> Human Papilloma virus

<400> 133
Glu Leu Thr Glu Val Phe Glu Phe Ala
1               5

<210> 134
<211> 10
<212> PRT
<213> Human Papilloma virus

<400> 134
Lys Leu Thr Asn Thr Gly Leu Tyr Asn Leu
1               5                   10

<210> 135
<211> 9
<212> PRT
<213> Human Papilloma virus

<400> 135
Leu Thr Asn Thr Gly Leu Tyr Asn Leu
1               5

<210> 136
<211> 10
<212> PRT
<213> Human Papilloma virus

<400> 136
Val Leu Glu Leu Thr Glu Val Phe Glu Phe
1               5                   10

<210> 137
<211> 9
<212> PRT
<213> Human Papilloma virus

<400> 137

Ser Val Tyr Gly Asp Thr Leu Glu Lys
1               5

<210> 138
<211> 10
<212> PRT
<213> Human Papilloma virus

<400> 138
Leu Leu Ile Arg Cys Leu Arg Cys Gln Lys
1               5                   10

<210> 139
<211> 10
<212> PRT
<213> Human Papilloma virus

<400> 139
Cys Val Tyr Cys Lys Thr Val Leu Glu Leu
1               5                   10

<210> 140
<211> 9
<212> PRT
<213> Human Papilloma virus

<400> 140
Val Tyr Cys Lys Thr Val Leu Glu Leu
1               5

<210> 141
<211> 9
<212> PRT
<213> Human Papilloma virus

<400> 141
Val Tyr Gly Asp Thr Leu Glu Lys Leu
1               5

<210> 142
<211> 10
<212> PRT
<213> Human Papilloma virus

<400> 142
Leu Thr Asn Thr Gly Leu Tyr Asn Leu Leu
1               5                   10

<210> 143
<211> 10
<212> PRT
<213> Human Papilloma virus

<400> 143
His Leu Glu Pro Gln Asn Glu Ile Pro Val
1               5                   10

<210> 144
<211> 9
<212> PRT
<213> Human Papilloma virus

<400> 144
Thr Leu Gln Asp Ile Val Leu His Leu
1               5

<210> 145
<211> 10

<212> PRT
<213> Human Papilloma virus

<400> 145
Ala Thr Leu Gln Asp Ile Val Leu His Leu
1               5                   10

<210> 146
<211> 10
<212> PRT
<213> Human Papilloma virus

<400> 146
Gln Leu Phe Leu Asn Thr Leu Ser Phe Val
1               5                   10

<210> 147
<211> 10
<212> PRT
<213> Human Papilloma virus

<400> 147
Met Leu Cys Met Cys Cys Lys Cys Glu Ala
1               5                   10

<210> 148
<211> 10
<212> PRT
<213> Human Papilloma virus

<400> 148
Cys Met Cys Cys Lys Cys Glu Ala Arg Ile
1               5                   10

<210> 149
<211> 9
<212> PRT
<213> Human Papilloma virus

<400> 149
Phe Gln Gln Leu Phe Leu Asn Thr Leu
1               5

<210> 150
<211> 9
<212> PRT
<213> Human Papilloma virus

<400> 150
Thr Leu Ser Phe Val Cys Pro Trp Cys
1               5

<210> 151
<211> 10
<212> PRT
<213> Human Papilloma virus

<400> 151
Ala Phe Gln Gln Leu Phe Leu Asn Thr Leu
1               5                   10

<210> 152
<211> 42
<212> PRT
<213> Human Papilloma virus

<400> 152
Arg Arg Pro Tyr Lys Leu Pro Asp Leu Cys Thr Glu Leu Asn Thr Ser

1                   5                   10                  15
Leu Gln Asp Ile Glu Ile Thr Cys Val Tyr Cys Lys Thr Val Leu Glu
                20                  25                  30
Leu Thr Glu Val Phe Glu Phe Ala Phe Lys
            35                  40

<210> 153
<211> 27
<212> PRT
<213> Human Papilloma virus

<400> 153
Ser Val Tyr Gly Asp Thr Leu Glu Lys Leu Thr Asn Thr Gly Leu Tyr
  1               5                  10                  15
Asn Leu Leu Ile Arg Cys Leu Arg Cys Gln Lys
                20                  25

<210> 154
<211> 19
<212> PRT
<213> Human Papilloma virus

<400> 154
Lys Ala Thr Leu Gln Asp Ile Val Leu His Leu Glu Pro Gln Asn Glu
  1               5                  10                  15
Ile Pro Val

<210> 155
<211> 14
<212> PRT
<213> Human Papilloma virus

<400> 155
His Thr Met Leu Cys Met Cys Cys Lys Cys Glu Ala Arg Ile
  1               5                  10

<210> 156
<211> 17
<212> PRT
<213> Human Papilloma virus

<400> 156
Ala Phe Gln Gln Leu Phe Leu Asn Thr Leu Ser Phe Val Cys Pro Trp
  1               5                  10                  15
Cys

<210> 157
<211> 236
<212> PRT
<213> Artificial Sequence

<220>
<223> Artificial fusion sequence

<400> 157
Ala Met Phe Gln Asp Pro Gln Glu Arg Pro Arg Lys Leu Pro Gln Leu
  1               5                  10                  15
Cys Thr Glu Leu Leu Leu Arg Arg Glu Val Tyr Asp Phe Ala Phe Arg
                20                  25                  30
Asp Leu Cys Ile Val Tyr Arg Asp Gly Asn Pro Tyr Lys Ile Ser Glu
            35                  40                  45
Tyr Arg His Tyr Cys Tyr Ser Leu Tyr Gly Thr Thr Leu Glu Gln Gln
        50                  55                  60
Tyr Asn Lys Thr Leu His Glu Tyr Met Leu Asp Leu Gln Pro Glu Thr
 65                  70                  75                  80
Thr Asp Leu Tyr Ser Tyr Gln Ala Glu Pro Asp Arg Ala His Tyr Asn

85                  90                  95
Ile Val Thr Phe Leu Leu Met Gly Thr Leu Gly Ile Val Cys Pro Ile
            100                 105                 110
Cys Ser Gln Lys Pro Arg Arg Pro Tyr Lys Leu Pro Asp Leu Cys Thr
        115                 120                 125
Glu Leu Asn Thr Ser Leu Gln Asp Ile Glu Ile Thr Cys Val Tyr Cys
    130                 135                 140
Lys Thr Val Leu Glu Leu Thr Glu Val Phe Glu Phe Ala Phe Lys Ser
145                 150                 155                 160
Val Tyr Gly Asp Thr Leu Glu Lys Leu Thr Asn Thr Gly Leu Tyr Asn
                165                 170                 175
Leu Leu Ile Arg Cys Leu Arg Cys Gln Lys Lys Ala Thr Leu Gln Asp
            180                 185                 190
Ile Val Leu His Leu Glu Pro Gln Asn Glu Ile Pro Val His Thr Met
        195                 200                 205
Leu Cys Met Cys Cys Lys Cys Glu Ala Arg Ile Ala Phe Gln Gln Leu
    210                 215                 220
Phe Leu Asn Thr Leu Ser Phe Val Cys Pro Trp Cys
225                 230                 235

<210> 158
<211> 237
<212> PRT
<213> Artificial Sequence

<220>
<223> Artificial fusion sequence

<400> 158
Met Ala Met Phe Gln Asp Pro Gln Glu Arg Pro Arg Lys Leu Pro Gln
  1               5                  10                  15
Leu Cys Thr Glu Leu Leu Leu Arg Arg Glu Val Tyr Asp Phe Ala Phe
            20                  25                  30
Arg Asp Leu Cys Ile Val Tyr Arg Asp Gly Asn Pro Tyr Lys Ile Ser
        35                  40                  45
Glu Tyr Arg His Tyr Cys Tyr Ser Leu Tyr Gly Thr Thr Leu Glu Gln
    50                  55                  60
Gln Tyr Asn Lys Thr Leu His Glu Tyr Met Leu Asp Leu Gln Pro Glu
 65                  70                  75                  80
Thr Thr Asp Leu Tyr Ser Tyr Gln Ala Glu Pro Asp Arg Ala His Tyr
                85                  90                  95
Asn Ile Val Thr Phe Leu Leu Met Gly Thr Leu Gly Ile Val Cys Pro
            100                 105                 110
Ile Cys Ser Gln Lys Pro Arg Arg Pro Tyr Lys Leu Pro Asp Leu Cys
        115                 120                 125
Thr Glu Leu Asn Thr Ser Leu Gln Asp Ile Glu Ile Thr Cys Val Tyr
    130                 135                 140
Cys Lys Thr Val Leu Glu Leu Thr Glu Val Phe Glu Phe Ala Phe Lys
145                 150                 155                 160
Ser Val Tyr Gly Asp Thr Leu Glu Lys Leu Thr Asn Thr Gly Leu Tyr
                165                 170                 175
Asn Leu Leu Ile Arg Cys Leu Arg Cys Gln Lys Lys Ala Thr Leu Gln
            180                 185                 190
Asp Ile Val Leu His Leu Glu Pro Gln Asn Glu Ile Pro Val His Thr
        195                 200                 205
Met Leu Cys Met Cys Cys Lys Cys Glu Ala Arg Ile Ala Phe Gln Gln
    210                 215                 220
Leu Phe Leu Asn Thr Leu Ser Phe Val Cys Pro Trp Cys
225                 230                 235

<210> 159
<211> 119
<212> PRT
<213> Artificial Sequence

<220>
<223> Artificial fusion sequence

<400> 159
Arg Arg Pro Tyr Lys Leu Pro Asp Leu Cys Thr Glu Leu Asn Thr Ser
1               5                   10                  15
Leu Gln Asp Ile Glu Ile Thr Cys Val Tyr Cys Lys Thr Val Leu Glu
            20                  25                  30
Leu Thr Glu Val Phe Glu Phe Ala Phe Lys Ser Val Tyr Gly Asp Thr
        35                  40                  45
Leu Glu Lys Leu Thr Asn Thr Gly Leu Tyr Asn Leu Leu Ile Arg Cys
    50                  55                  60
Leu Arg Cys Gln Lys Lys Ala Thr Leu Gln Asp Ile Val Leu His Leu
65                  70                  75                  80
Glu Pro Gln Asn Glu Ile Pro Val His Thr Met Leu Cys Met Cys Cys
                85                  90                  95
Lys Cys Glu Ala Arg Ile Ala Phe Gln Gln Leu Phe Leu Asn Thr Leu
            100                 105                 110
Ser Phe Val Cys Pro Trp Cys
        115

<210> 160
<211> 261
<212> PRT
<213> Artificial Sequence

<220>
<223> Artificial fusion sequence

<400> 160
Met Ala Ile Ser Gly Val Pro Val Leu Gly Phe Phe Ile Ile Ala Val
1               5                   10                  15
Leu Met Ser Ala Gln Glu Ser Trp Ala Ala Met Phe Gln Asp Pro Gln
            20                  25                  30
Glu Arg Pro Arg Lys Leu Pro Gln Leu Cys Thr Glu Leu Leu Leu Arg
        35                  40                  45
Arg Glu Val Tyr Asp Phe Ala Phe Arg Asp Leu Cys Ile Val Tyr Arg
    50                  55                  60
Asp Gly Asn Pro Tyr Lys Ile Ser Glu Tyr Arg His Tyr Cys Tyr Ser
65                  70                  75                  80
Leu Tyr Gly Thr Thr Leu Glu Gln Gln Tyr Asn Lys Thr Leu His Glu
                85                  90                  95
Tyr Met Leu Asp Leu Gln Pro Glu Thr Thr Asp Leu Tyr Ser Tyr Gln
            100                 105                 110
Ala Glu Pro Asp Arg Ala His Tyr Asn Ile Val Thr Phe Leu Leu Met
        115                 120                 125
Gly Thr Leu Gly Ile Val Cys Pro Ile Cys Ser Gln Lys Pro Arg Arg
    130                 135                 140
Pro Tyr Lys Leu Pro Asp Leu Cys Thr Glu Leu Asn Thr Ser Leu Gln
145                 150                 155                 160
Asp Ile Glu Ile Thr Cys Val Tyr Cys Lys Thr Val Leu Glu Leu Thr
                165                 170                 175
Glu Val Phe Glu Phe Ala Phe Lys Ser Val Tyr Gly Asp Thr Leu Glu
            180                 185                 190
Lys Leu Thr Asn Thr Gly Leu Tyr Asn Leu Leu Ile Arg Cys Leu Arg
        195                 200                 205
Cys Gln Lys Lys Ala Thr Leu Gln Asp Ile Val Leu His Leu Glu Pro
    210                 215                 220
Gln Asn Glu Ile Pro Val His Thr Met Leu Cys Met Cys Cys Lys Cys
225                 230                 235                 240
Glu Ala Arg Ile Ala Phe Gln Gln Leu Phe Leu Asn Thr Leu Ser Phe
                245                 250                 255
Val Cys Pro Trp Cys
            260

<210> 161
<211> 340
<212> DNA
<213> Artificial Sequence

<220>

<223> Artificial fusion sequence

<400> 161
atggctatgt tccaggaccc acaggaacgc ccacgcaaac tgccacagct gtgcaccgag      60
ctcctcctgc gccgcgaggt gtatgacttc gcttttcgcg acctgtgcat cgtctaccgc     120
gatggcaacc catataagat ctccgagtac cgccactact gctattccct gtatggcacc     180
accctggagc agcagtacaa caagaccctg cacgagtaca tgctggatct gcagccagag     240
accaccgatc tgtactccta tcaggctgag ccagatcgcg ctcactataa catcgtgacc     300
ttcctgctga tgggcaccct gggcatcgtg tgcccaatct                           340

<210> 162
<211> 786
<212> DNA
<213> Artificial Sequence

<220>
<223> Artificial fusion sequence

<400> 162
atggctatct ccggcgtgcc agtgctgggc ttcttcatca tcgctgtgct gatgtccgct      60
caggagtcct gggctgctat gttccaggac ccacaggaac gcccacgcaa actgccacag     120
ctgtgcaccg agctcctcct gcgccgcgag gtgtatgact tcgcttttcg cgacctgtgc     180
atcgtctacc gcgatggcaa cccatataag atctccgagt accgccacta ctgctattcc     240
ctgtatggca ccaccctgga gcagcagtac aacaagaccc tgcacgagta catgctggat     300
ctgcagccag agaccaccga tctgtactcc tatcaggctg agccagatcg cgctcactat     360
aacatcgtga ccttcctgct gatgggcacc ctgggcatcg tgtgcccaat ctgctcccag     420
aagccccggc gaccctacaa gctacctgat ctgtgcacgg aactgaacac ttcactgcaa     480
gacatagaaa taacctgtgt atattgcaag acagtattgg aacttacaga ggtatttgaa     540
tttgcattta aatctgtgta tggagacaca ttggaaaaac taactaacac tgggttatac     600
aatttattaa taaggtgcct gcggtgccag aaaaaggcaa cattgcaaga cattgtattg     660
catttagagc ccaaaatgaa aattccggtt cacacaatgt tgtgtatgtg ttgtaagtgt     720
gaagccagaa ttgcattcca gcagctgttt ctgaacaccc tgtcctttgt gtgtccgtgg     780
tgttaa                                                                786

<210> 163
<211> 9
<212> PRT
<213> Human Papilloma virus

<400> 163
Glu Val Tyr Asp Phe Ala Phe Arg Asp
1               5

## Description

### Field of the Invention

This invention relates generally to vaccines, and in particular to nucleic acid vaccines.

### Background of the Invention

Papilloma viruses are non-enveloped DNA viruses with a double stranded circular genome of approximately 9,000 base pairs. Over 75 types of HPV have been typed at the DNA level, and these can be broadly grouped into families on the basis of their tissue tropism.

Histologic, molecular, and epidemiologic evidence have implicated some HPV strains in cervical dysplasia and cervical cancer. Many studies support the view that most moderate and severe cervical intraepithelial neoplasias (CIN) contain HPV DNA which is exclusively detected in the histologically abnormal epithelium of these lesions. Persistent infection with HPV is believed to be the predominant risk factor for development of cervical carcinoma. HPV DNA is readily found in episomal form within cells exhibiting a cytopathic effect, while the HPV DNA is found integrated within the chromosomes of cells associated with most high grade precancerous lesions and cancer. Approximately 23 HPV types are commonly found in anogenital screening programs, but only 10-15 are associated with progressive disease. Types 16 and 18 are commonly found in association with cervical dysplasia and cervical cancer.

Papilloma viruses contain nine open reading frames. HPV genes with transforming properties have been mapped to open reading frames E6 and E7. Substantial biochemical work has demonstrated that the HPV E6 protein inactivates the protein p53, whereas the E7 protein interferes with retinoblastoma (Rb) protein function. Since p53 and Rb are tumor-suppressor proteins that function as cell division inhibitors, their inactivation by E6 and E7 leads the cell to enter into S phase of the cell cycle. Expression of E6 and E7 is sufficient to immortalize some primary cell lines, and blocking E6 or E7 function has been shown to reverse the transformed state.

### Summary of the Invention

The invention is based in part on the discovery that polypeptide segments, each of which contains one or more antibody-binding or class I or class II MHC-binding epitopes, can be linked in tandem to form polyepitope polypeptides which are proteolytically processed in cells to release the epitopes. These hybrid polypeptides are useful for stimulating an immune response, particularly when expressed from a nucleic acid within an antigen-presenting cell (APC).

In one aspect, the invention features a nucleic acid encoding a hybrid polypeptide that can include, in any order, a first, second, and third segment, each of which is at least 11 amino acids in length. As used herein, a "segment" is an amino acid sequence which (a) corresponds to the sequence of a portion (i.e., fragment less than all) of a naturally occurring protein, and (b) contains one or more epitopes. For clarity, the term "segment" is used herein to denote a part of the hybrid polypeptide, while the term "portion" is used to denote the corresponding part of the naturally occurring protein. By "epitope" is meant a peptide which binds to the binding groove of an MHC class I or class II molecule or to the antigen-binding region of an antibody. A methionine codon is preferably included at the 5' end of this or any other coding sequence of the invention, to facilitate translation. In addition, the hybrid polypeptide can encode a targeting signal, as described in more detail below.

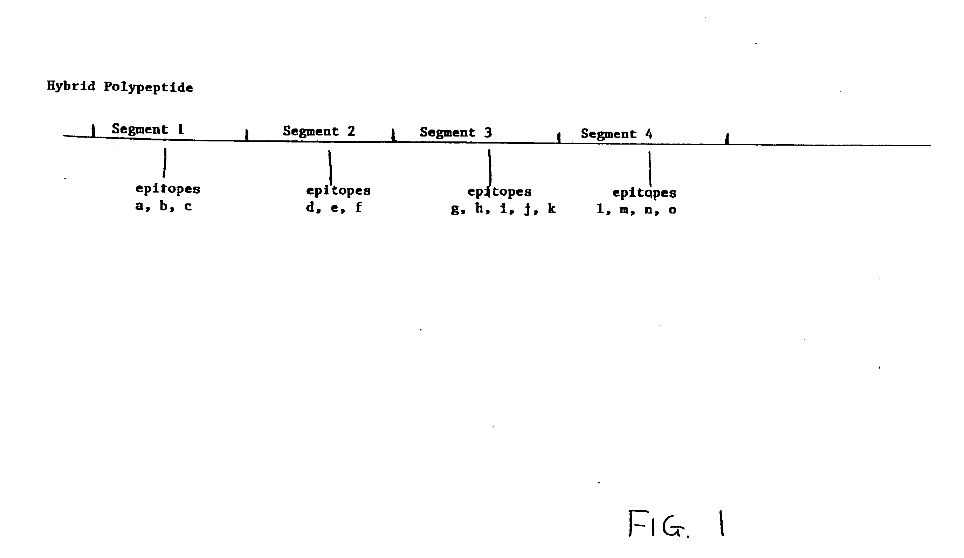

The relationship between segments and epitopes in the hybrid polypeptides of the invention is shown schematically in Fig. 1. The illustrated hybrid polypeptide is composed of segments 1, 2, 3, and 4, each of which separately corresponds to a portion of a naturally occurring protein. Segment 1 can be proteolytically processed within a cell to generate epitopes a, b, and c. Similarly, segment 2 can be proteolytically processed to generate epitopes d, e, and f; segment 3 can be processed to generate epitopes g, h, i, j, and k; and segment 4, upon processing, yields epitopes 1, m, n and o. Adjacent segments can be contiguous, or can be separated by a spacer amino acid or spacer peptide, as defined below.

In one embodiment, the first segment of the hybrid polypeptide of the invention has the amino acid sequence of a portion of (1) a naturally occurring protein of a pathogenic agent or (2) a naturally occurring tumor antigen (collectively referred to as "naturally occurring proteins"), is at least 11 amino acids in length, and contains at least two epitopes. The second segment has the amino acid sequence of a second portion of the same or a different naturally occurring protein, is at least 11 amino acids in length, and includes at least two epitopes which are different from the epitopes of the first segment. The third segment has the amino acid sequence of a third portion of the same or a different naturally occurring protein, is at least 11 amino acids in length, and includes at least two epitopes which are different from the epitopes of the first and second segments. When the first, second and third portions are from the same naturally occurring protein, they are preferably non-contiguous within that protein, and the sum of all three portions can constitute less than 70% of the full sequence of the naturally occurring protein. Alternatively, the first, second and third portions may be portions of two or three different naturally occurring proteins (e.g., two or three different tumor antigens, or two or three different naturally occurring proteins of one, two or three different pathogenic agents such as HPV). The polypeptide may optionally include additional segments, e.g., it can include at least 4, 5, 10, 15, 20, 25, 30, 40, 50, 60, 75, 90, or even 100 or more segments, each being a portion of a naturally-occurring protein of a pathogenic agent and/or of a naturally occurring tumor antigen which can be the same or different from the protein(s) from which the other segments are derived. Each of these segments can be at least 11 amino acids in length, and each contains at least one epitope (preferably two or more) different from the epitopes of the first, second, and third segments. At least one (preferably at least two or three) of the segments in the hybrid polypeptide may contain, e.g., 3, 4, 5, 6, 7, or even 10 or more epitopes, particularly class I or class II MHC-binding epitopes. Two, three, or more of the segments can be contiguous in the hybrid polypeptide: i.e., they are joined end-to-end, with no spacer between them. Alternatively, any two adjacent segments can be linked by a spacer amino acid or spacer peptide.

When the hybrid polypeptide is introduced into a cell, it is proteolytically processed into at least some of its constituent epitopes. At least some of the epitopes generated from the segments in the polypeptide can bind to MHC class I or MHC class II molecules present in the cell, though some of the epitopes may be specific for MHC class I or class II molecules present only on other cells. The epitopes may alternatively be B cell epitopes which elicit antibody-mediated immune responses upon binding to antibody receptors on the surface of a B cell.

A given segment within the hybrid polypeptide need not be any specified length, so long as it is sufficiently long to generate at least one epitope, e.g., 2, 3, 4, 5, or more epitopes, and is at least 11 amino acids in length. For example, a given segment can have a length of at least 12 amino acids, e.g., at least 13, 14, 15, 20, 25, 30, 40, or 50 amino acids. A given segment corresponds to a particular naturally occurring protein if any 11 (or more) consecutive amino acids of the segment are found in exactly the same order in a portion of the naturally occurring protein. The lengths of HPV strain 16 E6 and E7 proteins are 158 and 98 amino acids, respectively, and the lengths of the HPV strain 18 E6 and E7 proteins are 158 and 105 amino acids, respectively. The segment is preferably less than 100 amino acids (less than 95 amino acids for the HPV strain 16 E7 protein) and more preferably less than 70 or less than 50 amino acids (e.g., 20-50). In one embodiment, it is less than 15. The segments within the polyepitope polypeptide can be arranged in any order within the polypeptide. The hybrid polypeptide preferably does not contain a sequence identical to the sequence of either full length, intact E6 or full length, intact E7 protein from HPV strain 16 or 18. The sequences of these proteins can be found at the web sites (ftp://ftp-t10.lanl.gov/pub/papilloma/SWISS-PROT-files/Human-papilloma/HPV16.swp) and (ftp://ftp-t10.lanl.gov/pub/papilloma/SWISS-PROT-files/Human-papilloma/HPV18.swp) as viewed on December 7, 1999.

The segments can be derived from any tumor antigen or naturally occurring antigenic protein of a pathogenic agent. As used herein, "pathogenic agent" means a virus or microorganism that causes disease in a mammal. By "tumor antigen" is meant a protein or epitope which is expressed or in a tumor cell but not, or to a lesser degree, on a cell which is the non-tumor homolog of the tumor cell. Such tumor antigens frequently serve as markers for differentiating tumor cells from their normal counterparts. The tumor antigen can be, e.g., one or more of the tumor antigens listed in Table 1, such as a protein encoded by the Her2/neu gene, the prostate specific antigen gene, the melanoma antigen recognized by T cells (MART) gene, or the melanoma antigen gene (MAGE). Examples of proteins from pathogenic agents include proteins naturally expressed from the genome of a virus, e.g., a virus which chronically infects cells, such as human papillomavirus (HPV), human immunodeficiency virus (HIV), herpes simplex virus (HSV), hepatitis B virus (HBV), or hepatitis C virus (HCV); a bacterium, such as mycobacteria, Helicobacter spp. e.g., *Helicobacter pylori*, or *Chlamydia* spp.; a fungus; or a parasitic eukaryote, such as *Plasmodium* species.

A representative list of class I-binding epitopes of appropriate proteins, any of which could be included in the polyepitope polypeptides of the invention, is included in Table 2.

The hybrid polypeptide can contain a first epitope from an HPV protein and a second epitope that does not overlap with the first epitope and is from the same or a different HPV protein. ("Different HPV protein" can include a non-identical homolog of the first protein, derived from a different HPV strain than that from which the first protein was derived.) The first epitope binds to a first major histocompatibility complex (MHC) class I allotype and the second epitope binds to a second MHC class I allotype different from the first MHC class I allotype.

Because the hybrid polypeptide is made up of protein fragments from different proteins, or from non-contiguous fragments of a single protein, optionally linked by spacer amino acids or spacer sequences and optionally joined to a methionine residue or a targeting signal (see below), the sequence of this hybrid polypeptide is not identical to the amino acid sequence of either a naturally occurring protein or a fragment of a naturally occurring protein. One or more of the epitopes can be from, e.g., the HPV E6 or E7 protein, and the protein can be from, e.g., HPV strain 16, 18, 31, 33, 43, or 45. Thus, the hybrid polypeptide can include, e.g., one or more segments from each of the HPV strain 16 E6 and E7 proteins, and/or one or more segments from each of the HPV strain 18 E6 and E7 proteins. The hybrid polypeptide is at least 33 amino acids in length, and can be, e.g., at least 34, 35, 40, 44, 45, 50, 75, 100, 150, 200, 250, or 300 amino acids.

The MHC class I allotypes to which the epitopes in the hybrid polypeptide bind can be any human class I allotypes, e.g., HLA-A1, HLA-A2, HLA-A3, HLA-A11, and/or HLA-A24. A given epitope may be promiscuous, i.e., bind more than one allotype.

The hybrid polypeptide may also include a third epitope of an HPV protein. This epitope, which can be derived from the same or a different HPV protein as the first and/or second epitope, binds to a third MHC class I allotype different from the first and second MHC class I allotypes. It may, of course, also bind to the first and/or second MHC class I allotype, in addition to the third. The hybrid polypeptide may include, e.g., at least 4, 5, 6, 7, 8, 9, 10, 15, 25, 35, 40, 50, 60, 80, or even 100 or more MHC class I allotype-binding epitopes from one or more HPV proteins. Some of these epitopes may overlap.

**TABLE 1: Tumor Antigens**

| Cancer | Associated Antigen |
|---|---|
| Melanoma | BAGE 2-10 |
| Breast/Ovarian | c-ERB2 (Her2/neu) |
| Burkitt's lymphoma/Hodgkin's lymphoma | EBNA-1 |
| Burkitt's lymphoma/Hodgkin's lymphoma | EBNA-2 |
| Burkitt's lymphoma/Hodgkin's lymphoma | EBNA-3 |
| Burkitt's lymphoma/Hodgkin's lymphoma | EBNA-3A |
| Burkitt's lymphoma/Hodgkin's lymphoma | EBNA-3C |
| Burkitt's lymphoma/Hodgkin's lymphoma | EBNA-4 |
| Burkitt's lymphoma/Hodgkin's lymphoma | EBNA-6 |
| Burkitt's lymphoma/Hodgkin's lymphoma | EBV |
| Burkitt's lymphoma/Hodgkin's lymphoma | EBV LMP2A |
| Melanoma | GAGE-1 |
| Melanoma | gp75 |
| Cervical | HPV 16 E6 |
| Cervical | HPV 16 E7 |
| Cervical | HPV 18 E6 |
| Cervical | HPV 18 E7 |
| Melanoma | MAG |
| Melanoma | MAGE-1 |
| Melanoma | MAGE-2 |
| Melanoma | MAGE-3 |
| Melanoma | MAGE-4b |
| Melanoma | MAGE-5 |
| Melanoma | MAGE-6 |
| Melanoma | MART-1/Melan-A |
| PancreaticBreast/Ovarian | MUC-1 |
| Melanoma | MUM-1-B |
| Breast/Colorectal/Burkitt's lymphoma | p53 |
| Melanoma | Pmel 17(gp 100) |
| Prostate | PSA (Prostate Specific |
| Antigen) | |
| Melanoma | Tyrosinase |
| multiple | CEA (Carcinoembryonic |
| Antigen) | |
| lung | LRP (Lung Resistance Protein) |
| multiple | Bcl-2 |
| | Ki-67 |

**TABLE 2: Class I-associated Tumor and Pathogen Epitopes**

| Peptide | SEQ ID NO: | Source Protein |
|---|---|---|
| AARAVFLAL | 1 | BAGE 2-10 |
| YRPRPRRY | 2 | GAGE-1 9-16 |
| EADPTGHSY | 3 | MAGE-1 161-169 |
| SAYGEPRKL | 4 | MAGE-1 230-238 |
| EVDPIGHLY | 5 | MAGE-3 161-169 |
| FLWGPRALV | 6 | MAGE-3 271-279 |
| GIGILTV | 7 | MART-1 29-35 |
| ILTVILGV | 8 | MART-1 32-39 |
| STAPPAHGV | 9 | MUC-1 9-17 |
| EEKLIVVLF | 10 | MUM-1 261-269 |
| MLLAVLYCL | 11 | TYROSINASE 1-9 |
| SEIWRDIDF | 12 | TYROSINASE 192-200 |
| AFLPWHRLF | 13 | TYROSINASE 206-214 |
| YMNGTMSQV | 14 | TYROSINASE 369-376 |
| KTWGQYWQV | 15 | PMEL 17 (GP100) 154-162 |
| ITDQVPFSV | 16 | PMEL 17 (GP100) 209-217 |
| YLEPGPTVA | 17 | PMEL 17 (GP100) 280-288 |
| LLDGTATLRL | 18 | PMEL 17 (GP100) 476-485 |
| ELNEALELEK | 19 | p53 343-351 |
| STPPPGTRV | 20 | p53 149-157 |
| LLPENNVLSPL | 21 | p53 25-35 |
| LLGRNSFEV | 22 | p53 264-272 |
| RMPEAAPPV | 23 | p53 65-73 |
| KIFGSLAFL | 24 | HER-2/neu 369-377 |
| IISAVVGIL | 25 | HER-2/neu 654-662 |
| CLTSTVQLV | 26 | HER-2/neu 789-797 |
| YLEDVRLV | 27 | HER-2/neu 835-842 |
| VLVKSPNHV | 28 | HER-2/neu 851-859 |
| RFRELVSEFSRM | 29 | HER-2/neu 968-979 |
| LLRLSEPAEL | 30 | PSA 119-128 |
| DLPTQEPAL | 31 | PSA 136-144 |
| KLQCVD | 32 | PSA 166-171 |
| VLVASRGRAV | 33 | PSA 36-45 |
| VLVHPQWVL | 34 | PSA 49-57 |
| DMSLLKNRFL | 35 | PSA 98-107 |
| QWNSTAFHQ | 36 | HBV envelope 121-130 |
| VLQAGFF | 37 | HBV envelope 177-184 |
| LLLCLIFL | 38 | HBV envelope 250-257 |
| LLDYQGML | 39 | HBV envelope 260-267 |
| LLVPFV | 40 | HBV envelope 338-343 |
| SILSPFMPLL | 41 | HBV envelope 370-379 |
| PLLPIFFCL | 42 | HBV envelope 377-385 |
| ILSTLPETTV | 43 | HBV core 529-538 |
| FLPSDFFPSV | 44 | HBV core 47-56 |
| KLHLYSHPI | 45 | HBV polymerase 489-498 |
| ALMPLYACI | 46 | HBV polymerase 642-651 |
| HLYSHPIIL | 47 | HBV polym. 1076-1084 |
| FLLSLGIHL | 48 | HBV polym. 1147-1153 |
| HLLVGSSGL | 49 | HBV polymerase 43-51 |
| GLSRYVARL | 50 | HBV polymerase 455-463 |
| LLAQFTSAI | 51 | HBV polymerase 527-535 |
| YMDDVVLGA | 52 | HBV polymerase 551-559 |
| GLYSSTVPV | 53 | HBV polymerase 61-69 |
| NLSWL | 54 | HBV polymerase 996-1000 |
| KLPQLCTEL | 55 | HPV 16 E6 18-26 |
| LQTTIHDII | 56 | HPV 16 E6 26-34 |
| FAFRDLCIV | 57 | HPV 16 E6 52-60 |
| YMLDLQPET | 58 | HPV 16 E7 11-19 |
| TLHEYMLDL | 59 | HPV 16 E7 7-15 |
| LLMGTLGIV | 60 | HPV 16 E7 82-90 |
| TLGIVCPI | 61 | HPV 16 E7 86-93 |
| LLMGTLGIVCPI | 62 | HPV 16 E7 82-93 |

Unless otherwise specified, the epitopes in any of the hybrid polypeptides of the invention may overlap to some degree, e.g., the first epitope may overlap (i.e., share at least one amino acid residue, and share up to all but one residue) with the third epitope, or they may be non-overlapping.

The polypeptide may optionally include a targeting signal. A targeting signal is a peptide which directs intracellular transport or secretion of a peptide to which it is attached. The targeting signal can be at the amino terminus, e.g., a signal sequence, or carboxy terminus, or within the hybrid polypeptide, so long as it functions in that site.

The targeting signal can be any recognized signal sequence, e.g., a signal sequence from the adenovirus E3 protein. A preferred targeting signal is the signal peptide of HLA-DRα: Met Ala Ile Ser Gly Val Pro Val Leu Gly Phe Phe Ile Ile Ala Val Leu Met Ser Ala Gln Glu Ser Trp Ala (SEQ ID NO:63).

The targeting signal may optionally be modified to introduce an amino acid substitution at the junction(s) between the targeting signal and the adjacent segment(s) to promote cleavage of the targeting sequence from the epitopes by, e.g., a signal peptidase.

Any of the segments within the hybrid polypeptide may be separated from the others by a spacer amino acid or a spacer sequence.

The nucleic acid of the invention may encode a hybrid polypeptide including a first epitope and a second epitope, wherein the first epitope is from a first HPV protein and the second epitope is from a second HPV protein different from the first HPV protein, provided that the sequence of the second epitope does not occur within the first HPV protein, i.e., they are necessarily derived from different proteins. The different proteins can be from the same or different strains of HPV, e.g., types 16 and 18. The hybrid polypeptide can, of course, include additional epitopes from the same or different HPV proteins, or from other pathogens or tumor antigens.

Also within the invention is a nucleic acid encoding a hybrid polypeptide including a plurality (e.g., at least 2, 3, 4, 5, 6, 7, 8, 9, 10, 15, 20, or 30) MHC class I-binding epitopes from an HPV protein. The sequence of the entire hybrid polypeptide is not identical to the sequence of either a naturally occurring HPV protein or a fragment of a naturally occurring HPV protein, by virtue of sequence insertions, internal deletions, or substitutions, accomplished, e.g., by genetic engineering techniques.

The nucleic acid may encode a hybrid polypeptide that includes a plurality of HLA-binding epitopes from an HPV strain 16 E6 protein, a plurality of HLA-binding epitopes from an HPV strain 16 E7 protein, a plurality of HLA-binding epitopes from an HPV strain 18 E6 protein, and a plurality of epitopes from an HPV strain 18 E7 protein. Each plurality of epitopes can include an epitope selected from the group consisting of an HLA-A1-binding epitope, an HLA-A2-binding epitope, an HLA-A3-binding epitope, an HLA-A11-binding epitope, and an HLA-A24-binding epitope, and preferably at least two or three selected from this group. The members of each plurality of epitopes are different from the members of each of the other plurality of epitopes. Each plurality of epitopes may include at least two HLA-A1-binding epitopes, at least two HLA-A2-binding epitopes, at least two HLA-A3-binding epitopes, at least two HLA-A11-binding epitopes, and/or at least two HLA-A24-binding epitopes. A given plurality can be distributed on more than one segment.

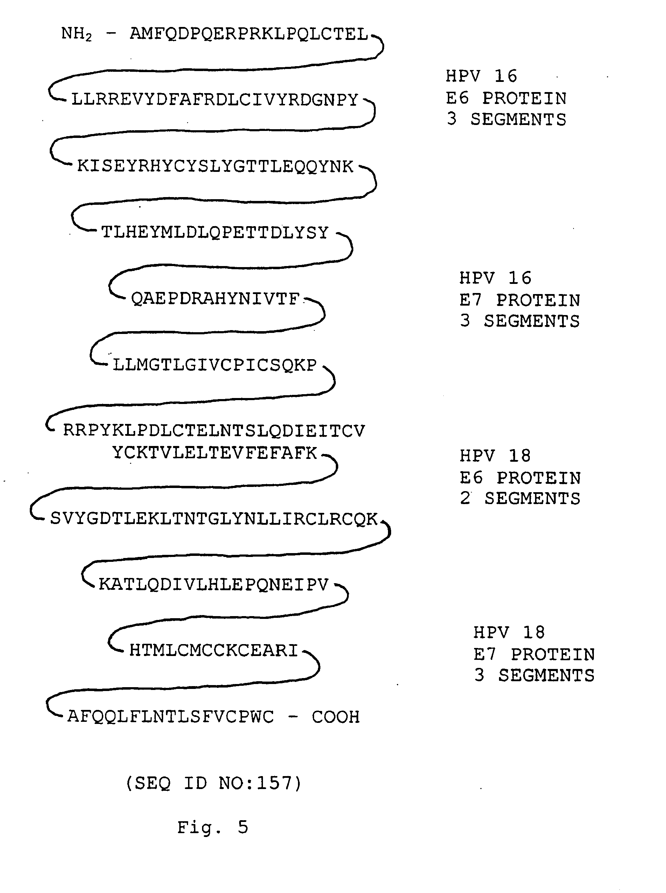

The invention also includes a nucleic acid encoding a hybrid polypeptide (SEQ ID NO:157) comprising any one or more of the peptide segments of Fig. 5. The polypeptide can thus include one or more of the segments
AMFQDPQERPRKLPQLCTEL (SEQ ID NO:64),
LLRREVYDFAFRDLCIVYRDGNPY (SEQ ID NO:65),
KISEYRHYCYSLYGTTLEQQYNK (SEQ ID NO:66),
TLHEYMLDLQPETTDLYSY (SEQ ID NO:67),
QAEPDRAHYNIVTF (SEQ ID NO:68),
LLMGTLGIVCPICSQKP (SEQ ID NO:69),
RRPYKLPDLCTELNTSLQDIEITCVYCKTVLELTEVFEFAFK (SEQ ID NO:152),
SVYGDTLEKLTNTGLYNLLIRCLRCQK (SEQ ID NO:153),
KATLQDIVLHLEPQNEIPV (SEQ ID NO:154),
HTMLCMCCKCEARI (SEQ ID NO:155), and
AFQQLFLNTLSFVCPWC (SEQ ID NO:156).

The segments can be processed to produce multiple epitopes. The hybrid polypeptide can include one or more of the peptide segments of Fig. 5, as well as additional HPV E6 or E7 sequence. Most preferably, the segment is less than 100, 70, 50, 40, 30, or 20 amino acids. The hybrid polypeptide preferably does not contain a sequence identical to the sequence of either full length, intact E6 or full length, intact E7 protein from HPV strain 16 or 18.

Also featured in the invention is a nucleic acid encoding a hybrid polypeptide which includes at least three non-overlapping segments of one or more proteins of an infectious agent, e.g., a virus such as HPV, a bacterium, or a eukaryotic parasite. Each segment includes at least three epitopes which bind to one or more of the HLA-A1, -A2, -A3, -A11, and -A24 allotypes; in some embodiments, at least three and preferably four or even all five of these allotypes bind to epitopes in the hybrid polypeptide. The sequence of the entire hybrid polypeptide is not identical to the sequence of a naturally occurring protein. The sequence of the entire hybrid polypeptide is also not identical to the sequence of a fragment of any naturally occurring protein, i.e., the polypeptide has to differ from the sequence of a naturally occurring protein by something other than mere truncation of the latter.

The nucleic acids described above can be provided in a plasmid, bacterial, or viral vector. When the nucleic acids are used *in vivo,* it is preferable to use plasmid vectors containing the above-described nucleic acids. The vector is preferably an expression vector which contains one or more regulatory sequences (which could include a promoter) which permit expression in a cell of interest. The regulatory sequence (s) are operatively linked to the sequence encoding the hybrid polypeptide, such that they drive expression of the latter.

Also within the invention is a cell into which has been introduced (e.g., by transfection or infection) the plasmid, bacterial, or viral vector, either transiently or stably. The cell can be, e.g., a B cell, dendritic cell (DC), Langerhans cell, or other antigen presenting cell (APC). The cell may be cultured or otherwise maintained under conditions permitting expression of the hybrid polypeptide from the nucleic acid, e.g., the plasmid, encoding it.

As used herein, "isolated DNA" covers both (1) a DNA the full sequence of which does not occur within any naturally occurring DNA, and (2) a DNA the full sequence of which does occur within a naturally occurring DNA, but which is free of the genes that flank that sequence in the genome of the organism in which that sequence naturally occurs. The term therefore includes a recombinant DNA incorporated into a vector, into an autonomously replicating plasmid or virus, or into the genomic DNA of a prokaryote or eukaryote. It also includes a separate molecule such as a cDNA, a genomic fragment, a fragment produced by polymerase chain reaction (PCR), or a restriction fragment.

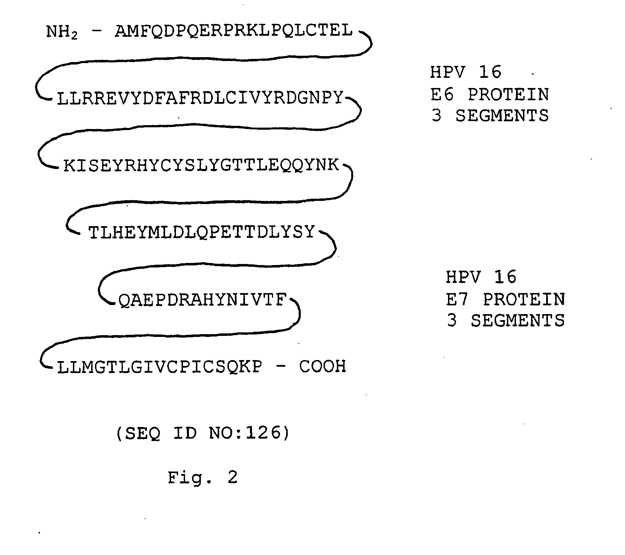

The invention further includes hybrid polypeptides encoded by the above-described nucleic acids. The hybrid polypeptides described herein may optionally include an amino terminal methionine residue. The hybrid polypeptides described herein also may optionally include a GLAG (or other monoclonal antibody determinant) or an antibody recognition site (e.g., a myc or his tag) located directly 3' of the last epitope coding sequence. Also included in the hybrid polypeptide are segments, as discussed above, which may include one or more of the amino acid sequences recited in Fig. 2 or Table 3, either singly, in tandem, or overlapping. At least some of the segments correspond to portions of one or more naturally occurring proteins. They may be separated by spacer amino acids or spacer peptides.

The hybrid polypeptide described herein can be a substantially pure polypeptide. "Substantially pure polypeptide" covers both (1) a polypeptide the full sequence of which is not identical to that of any naturally occurring polypeptide, and (2) a polypeptide which does have the sequence of a naturally occurring polypeptide, but which is separated from those components (proteins and other naturally-occurring organic molecules) which naturally accompany it in the biological context (e.g., cell) in which it naturally occurs. Typically, the polypeptide is substantially pure when it constitutes at least 60%, by weight, of the protein in the preparation. Preferably, at least 75%, more preferably at least 90%, and most preferably at least 99%, by weight, of the protein in preparation is the polypeptide.

The nucleic acids (e.g., viral, bacterial, or plasmid vectors) or hybrid polypeptides can optionally be provided in a microparticle that also includes a polymeric matrix. In preferred embodiments, the polymeric matrix consists essentially of poly-lactic acid (PLA), poly-glycolic acid (PGA), or a copolymer of poly-lactide-co-glycolide acid (PLGA). The microparticle preferably has a diameter of, e.g., 0.02 to 20 microns, or less than about 11 microns. A plurality of the microparticles preferably has an average diameter of, e.g., 0.02 to 20 microns, less than about 11 microns, or less than about 5 microns.

The nucleic acids and hybrid polypeptides described herein can alternatively be incorporated into liposomes or immune-stimulating complexes (ISCOMS) or delivered with naturally occurring polymers, synthetic polymers, biopolymers, cationic lipids, condensing agents, dendrimers, other biomaterials, oil-containing adjuvants, and other adjuvants such as QS21 or saponin. The nucleic acids and hybrid polypeptides described herein may alternatively be administered using any other suitable delivery vehicle known in the art, or can be delivered without a delivery vehicle (other than aqueous solution), e.g., "naked DNA".

The invention in addition includes a therapeutic composition containing the above-described nucleic acids or polypeptides, a pharmaceutically acceptable carrier, and optionally, one of the above-discussed delivery vehicles.

Also provided is a method of eliciting an immune response (e.g., an antibody response or a cellular immune response, including an MHC class I-mediated or class II-mediated immune response) in a mammal by administering the above-described nucleic acids or hybrid polypeptides to the mammal. Preferably, the mammal bears at least one MHC class I or II allotype which binds to an epitope derived from the polyepitope polypeptide. The mammal can be, e.g., a human, non-human primate, dog, cat, rabbit, cow, horse, sheep, pig, goat, mouse, rat, guinea pig, or hamster.

When at least one of the epitopes is an HPV epitope, appropriate subjects for the method include, e.g., a human who suffers from, or is at risk of, condyloma, e.g., exophytic condyloma, flat condyloma, cervical cancer, other HPV-associated-cancers diseases or conditions, respiratory papilloma, conjunctival papilloma, genital-tract or anal-tract HPV infection, or cervical dysplasia. The nucleic acid or hybrid polypeptide, or delivery vehicle containing one or more of these compositions, can be administered directly to a mucosal tissue, e.g., vaginal, nasal, lower respiratory, ocular, or applied to gastrointestinal (e.g., rectal, cervical, or dermal) tissue, of the mammal. Alternatively, the nucleic acid or hybrid polypeptide, or delivery vehicle containing the same, can be administered systemically: for example, intravenously, intramuscularly, intradermally, orally, subcutaneously, intraarterially, intraperitoneally, or intrathecally.

By "spacer amino acid" is meant a single residue inserted between two neighboring segments ("A" and "B", in that order) in a polypeptide of the invention, where the residue is different from the amino acid which flanks the carboxy terminus of A and also is different from the amino acid which flanks the amino terminus of B in the respective full length proteins from which A and B were derived ("X" and "Y", respectively). Thus, the spacer amino acid forms a point of discontinuity from the X-derived sequence of A and the Y-derived sequence of B, in the polypeptide of the invention. Typically, the amino acid will be one of the twenty naturally occurring amino acids, e.g., Ala, Leu, Ile, or Gly, and in general can be any amino acid except (1) the one that naturally flanks the carboxy terminus of A in protein X, and (2) the one that naturally flanks the amino terminus of B in protein Y.

By "spacer sequence" is meant a sequence of two or more amino acid inserted between two neighboring segments, e.g., "A" and "B", in a polypeptide of the invention. The sequence of the spacer is different from the sequences which flank the carboxy terminus of A and the amino terminus of B in the respective full length proteins ("X" and "Y") from which A and B were derived. Thus, the spacer sequence forms a point of discontinuity from both the X-derived sequence of A and the Y-derived sequence of B in the polypeptide of the invention.

Examples of spacer sequences include Ala Ala, Ala Leu, Leu Leu, Leu Ala, Leu Ile, Ala Ala Ala, Ala Gly Leu, Phe Ile Ile, etc. Generally, the spacer sequence will include nonpolar amino acids, though polar residues such as Glu, Gln, Ser, His, and Asn could also be present, particularly for spacer sequences longer than three residues. The only outer limit on the total length and nature of each spacer sequence derives from considerations of ease of synthesis, proteolytic processing, and manipulation of the polypeptide and/or nucleic acid. It is generally unnecessary and probably undesirable to use spacer sequences longer than about four or five residues, though they could be, for example, up to 6, 8, 10, 15, 20, 30, or 50 residues. Of course, they could be even longer than 50 residues.

Spacer amino acids and spacer sequences are useful for promoting processing to release epitopes. The spacers are typically removed from the polypeptide by proteolytic processing in the cell, along with any sequence between epitopes within a given segment. This leaves the epitopes intact for binding to MHC molecules or (upon secretion from the cell) antibodies. Occasionally a spacer amino acid or part of a spacer sequence will remain attached to an epitope through incomplete processing. This generally will have little or no effect on binding to the MHC molecule.

An advantage of the invention is that it permits delivery of MHC class I or class II-binding epitopes from polypeptides having only a partial sequence of a protein of a pathogen or tumor antigen. Thus, problems associated with interference of antigen presentation by viral proteins, or deleterious effects seen in over-expression of particular viral proteins or tumor antigens, are avoided.

Another advantage of the invention is that the nucleic acid sequences, or delivery vehicles containing the nucleic acid sequences, may promote associated immune responses, e.g., IL-12 and γ-interferon (IFN) release from macrophages, NK cells, and T cells. For instance, phagocytosis of microspheres containing DNA in general can promote TNF and IFN secretion by human APC.

A further advantage of the invention is that the assortment of epitopes within the polyepitope polypeptides described herein increases the likelihood that at least one epitope will be presented by each of a variety of HLA allotypes. This allows for immunization of a population of individuals polymorphic at the HLA locus, using a single hybrid polypeptide or nucleic acid encoding the polypeptide. The hybrid polypeptide can be specific for a particular pathogen, including two or more strains of the same pathogen, or can contain epitopes derived from two or more pathogens. Alternatively or in addition, the hybrid polypeptide can be specific for a particular tumor antigen, or can contain epitopes derived from two or more tumor antigens.

Unless otherwise defined, all technical and scientific terms used herein have the same meaning as commonly understood by one of ordinary skill in the art to which this invention belongs. Suitable methods and materials are described below, although methods and materials similar or equivalent to those described herein can also be used in the practice or testing of the present invention. All publications, patent applications, patents, and other references mentioned herein are incorporated by reference in their entirety. In case of conflict, the present specification, including definitions, will control. In addition, the materials, methods, and examples are illustrative only and not intended to be limiting.

Other features and advantages of the invention will be apparent from the following detailed description, and from the claims.

### Brief Description of the Drawings

Fig. 1 is a schematic drawing of a polyepitope polypeptide which includes four segments, each segment including multiple epitopes.
Fig. 2 is a schematic drawing of a polyepitope polypeptide including the amino acid sequences of segments derived from the HPV strain 16 E6 and E7 proteins.
Fig. 3 is a graph illustrating the results of an *in vitro* assay in which human T cells stimulated with the peptide HPV 16E7 44-52 were tested against HLA-A1/A3-expressing targets infected with either wildtype vaccinia vector (open circles) or vaccinia vector encoding HPV 16E7 44-52 as part of an HPV 16 E6/E7 polyepitope polypeptide.
Fig. 4 is a schematic drawing of a polyepitope polypeptide including the amino acid sequences of segments derived from the HPV strain 18 E6 and E7 proteins.
Fig. 5 is a schematic drawing of a polyepitope polypeptide including the amino acid sequences of segments derived from the E6 and E7 proteins of HPV strains 16 and 18.
Fig. 6 is a graph illustrating the results of an *in vitro* assay in which T cells from DNA-immunized mice were expanded *in vitro* by exposure to peptide 124 or peptide 272 and subsequently tested against target cells infected with wildtype vaccinia vector, vaccinia vector encoding HPV Dra 16/18, or vaccinia vector encoding HPV 16/18.

### Detailed Description

The present invention provides nucleic acids encoding polyepitope polypeptides which include multiple immunogenic segments, each segment containing one or more MHC class I or II-restricted epitopes, or both. Nucleic acids and polypeptides of the invention are useful for generating or enhancing prophylactic or therapeutic immune responses against pathogens, tumors, or autoimmune disease in a population of individuals having diverse MHC allotypes. In addition, the nucleic acids and polypeptides of the invention are also useful as positive controls in T cell stimulation assays *in vitro,* and as tools to understand processing of epitopes within cells.

One or more of the segments present within the polyepitope polypeptide can be processed into multiple distinct epitopes to form either MHC class I-binding epitopes, which are typically associated with cytotoxic T cell (CTL) immune responses, or MHC class II-binding epitopes, which are typically associated with helper T cell immune responses. Other epitopes can be B cell epitopes which stimulate production of epitope-specific antibodies.

Included in the invention are nucleic acids encoding polyepitope polypeptides useful for preventing or treating HPV-associated infections, particularly infections associated with warts, cervical or anal dysplasia, cervical cancer, and other HPV-associated cancers. For example, the polypeptides and nucleic acids of the invention can be used as vaccines prophylactically or therapeutically in subjects known to be infected by HPV, suspected of being infected by HPV, or likely to become infected by HPV. Other suitable subjects include those displaying symptoms of, or likely to develop, HPV-associated conditions. The immunogenic polypeptides, and nucleic acids encoding these polypeptides, can also be used as vaccines for preventing or treating conditions associated with infections of HPV strain 16, e.g., bowenoid papulosis, anal dysplasia, respiratory or conjunctival papillomas, cervical dysplasia, cervical cancer, vulval cancer, or prostate cancer. They can also be used to treat conditions associated with other HPV strains, especially those associated with HPV strains 6, 11, 18, 31, 33, 35, and 45. These conditions include, e.g., exophytic condyloma (HPV strains 6 and 11), flat condyloma, especially of the cervix (HPV strains 6, 11, 16, 18, and 31), giant condyloma (HPV strains 6 and 11), cervical cancer (HPV strains 18, 31, and 33, in addition to HPV strain 16), respiratory and conjunctival papillomas (HPV 6 and 11), and infection with genital-tract HPVs (HPV 6, 11, 16, 18, 31, 33, and 35).

### Identification of MHC-binding epitopes from HPV proteins

For an epitope to generate effective cytotoxic T cell (CTL) responses, it must bind to an MHC molecule on an antigen-presenting cell (APC), and the resulting receptor-ligand complex must be recognized by a T cell receptor expressed on the CTL. Epitopes which bind to a specific MHC allele can be identified by first synthesizing a series of overlapping peptide fragments from a protein of interest, e.g., an HPV protein such as HPV E6 or E7, and testing the peptides in art-recognized binding studies with a radiolabeled peptide known to bind to the MHC allele. If a test peptide demonstrates specific binding to an MHC allele as measured by, for example, competition with the radiolabeled test peptide (i.e., it is an epitope), the epitope can be combined with additional epitopes (overlapping or adjacent) to produce or define a segment.

Alternatively, epitopes can be identified by refolding soluble MHC molecules in the presence of radiolabeled β2-microglobulin and a test peptide. The complete complex will refold and produce a receptor of the correct size. β2-microglobulin dissociates from the complex at a measurable rate that is directly correlated with the binding affinity of the test peptide (Garboczi et al., Proc. Nat. Acad. Sci. USA 89:3429-33, 1992; Parker et al., J. Biol. Chem. 267:5451-5459, 1992; and Parker et al., J. Immunol. 149: 1896-1904, 1992). Analysis of this type of data has resulted in an algorithm that predicts the dissociation times of a given test peptide for an HLA-A2 receptor (Parker et al., J. Immunol. 152:163-175, 1994). Fast dissociation has been correlated with low affinity, and slow dissociation with high affinity. This algorithm has been expanded and is available for predicting binding affinity of epitopes for the HLA-A allotypes, -A1, -A2, -A3, -A11, and -A24. The algorithm can be found at the web site (http://wwwbimas.dcrt.nih.gov/molbio/hla_bind/index.html). Most of the HPV 16 E6 and E7 epitopes that are known in the art to bind one of the five listed HLA-A receptors are predicted by this algorithm to cause β2-microglobulin dissociation times of greater than 2 h.

Epitope binding can also be determined using previously identified epitopes derived from HPV 16 E6 and E7 (Kast et al., J. Immunol. 152:3904-12, 1994). Additional binding studies on other HPV putative epitopes, e.g., HPV 18 E6- and E7- derived epitopes, are performed using overlapping 8, 9, or 10-mers from the HPV 18 E6 and E7 proteins in binding assays involving each of the five HLA-A allotypes, using one of the above-described protocols.

Alternatively, epitopes may be identified by identifying MHC class I or class II-binding peptides using techniques described in e.g., U.S. Pat. No. 5,827,516, and USSN 09/372,380, herein incorporated by reference.

### T cell assays for epitopes

Epitopes which bind *in vitro* to MHC molecules as described above can be analyzed for their effectiveness at stimulating human T cell-responses in an *in vitro* immunization assay. The assay has been used previously to identify human and murine T cell-responsive epitopes, including several that are derived from HPV proteins (Alexander et al., Amer. J. Obstet. and Gynecol. 175:1586-1593, 1996; Tarpey et al., Immunology 81:222-27, 1994). These assays have also been used to generate large numbers of specific CTL for immunotherapy (Tsai et al., Crit. Rev. Immunol. 18:65-75, 1998). To ensure reliability, it is desirable to perform the first round of T cell stimulation in the presence of dendritic cells (DCs) pulsed with the test peptide. Moreover, inclusion of IL-10 during the stimulation may suppress the non-specific responses that may sometimes arise during culture of the cells. T cell activation may then be examined using an ELISA assay to measure γ-IFN secretion, or by use of FACS to determine the increase in CD8+, CD16- cells containing γ-IFN by tricolor analysis. Alternatively, T cell activation can be measured using a ⁵¹Cr release CTL assay or a tetramer-based assay.

It is possible that not every individual with a given allotype will respond to a particular epitope. For example, one individual whose cells bear the HLABA2 allotype may respond to a given epitope, whereas a second such individual may not. One reason for this differential responsiveness may be that those individuals who have been infected with HPV previously have a higher precursor T cell frequency than a naive individual. Another may be related to differences in the T cell repertoire between the two individuals. To overcome this difficulty, T cells from two donors, and even more preferably three donors, for each HLA allotype can be tested. For the more common alleles (i.e., HLA-A2 and BA3) up to four donors are preferably tested.

Each epitope is tested initially with cells from one donor. If an epitope does not stimulate a T cell response using cells of the first donor, it is tested again with cells from a second donor, and then a third donor. If the epitope does not demonstrate T cell reactivity after two or three attempts, it is preferably not represented in the polypeptides of the invention.

Altering the method by which the *in vitro* presentation of antigen is performed may enhance analysis. An initial stimulation of T cells with DCs is typically part of the *in vitro* immunization. To enhance the immunization, DCs can be added at each round of stimulation to ensure adequate antigen presentation and T cell stimulation, e.g., using previously generated and subsequently frozen DCs. Leukopaks or blood samples can be obtained from individuals with high grade squamous intraepithelial lesions (HSIL) or cervical cancer. These individuals may have been primed *in vivo* and have increased numbers of T cells in their blood.

Alternatively, the epitopes can be selected for inclusion in the construct based solely on their binding affinity to HLA molecules, or identified based upon analysis of naturally processed peptides, as described herein and in Chicz et al., J. Exp. Med. 178:27-47, 1993, and U.S. Patent 5,827,516.

The amino acid sequence of a hybrid polypeptide (SEQ ID NO:126) based on sequences found in the HPV strain 16 E6 and strain 16 E7 proteins is shown in Fig. 2. The hybrid polypeptide is created by fusion of fragments of the E6 protein (amino acids 7-26 (SEQ ID NO:64), 44-67 (SEQ ID NO:65), and 79-101 (SEQ ID NO:66)) and the E7 protein (amino acids 7-25 (SEQ ID NO:67), 44-57 (SEQ ID NO:68), and 82-98 (SEQ ID NO:69)). To prevent retinoblastoma (Rb) protein binding to the hybrid polypeptide, amino acids 26 and 27 of the E7 protein are not included. In addition, the cysteine at amino acid 24 has been converted to a serine residue. In the parlance defined above, this renders that serine residue, together with residue 25, a "spacer sequence" between segments corresponding to E7 protein residues 7-23 and 44-57, respectively.

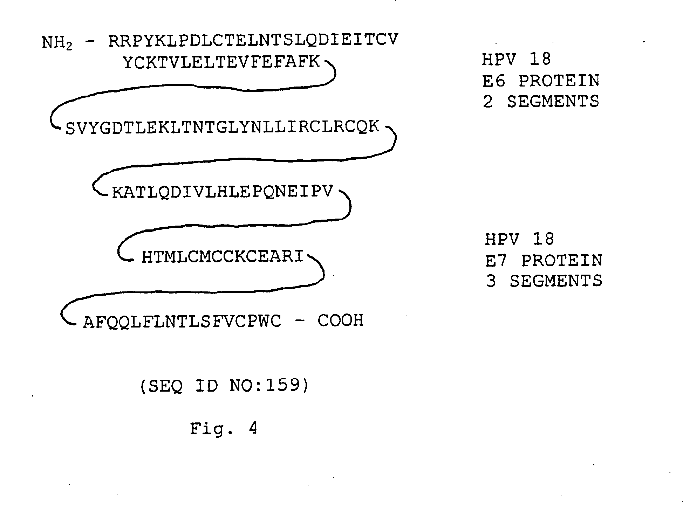

The amino acid sequence of a second hybrid polypeptide (SEQ ID NO:159) based on sequences found in the HPV strain 18 E6 and strain 18 E7 proteins is shown in Fig. 4. The hybrid polypeptide is created by fusion of fragments of the E6 protein (amino acids 9-50 (SEQ ID NO:152) and 84-110 (SEQ ID NO:153)), and the E7 protein (amino acids 5-23 (SEQ ID NO:154), 59-72 (SEQ ID NO:155), and 85-101 (SEQ ID NO:156)).

The amino acid sequence of a third hybrid polypeptide (SEQ ID NO:157) based on sequences found in the E6 and E7 proteins of HPV strains 16 and 18 is shown in Fig. 5. The hybrid polypeptide is created by fusion of fragments of the HPV strain 16 E6 protein (amino acids 7-26 (SEQ ID NO:64), 44-67 (SEQ ID NO:65), and 79-101 (SEQ ID NO:66)), the HPV strain 16 E7 protein (amino acids 7-25 (SEQ ID NO:67), 44-57 (SEQ ID NO:68), and 82-98 (SEQ ID NO:69)), the HPV strain 18 E6 protein (amino acids 9-50 (SEQ ID NO:152) and 84-110 (SEQ ID NO:153)), and the HPV strain 18 E7 protein (amino acids 5-23 (SEQ ID NO:154), 59-72 (SEQ ID NO:155), and 85-101 (SEQ ID NO:156)). As described above with respect to the hybrid polypeptide of SEQ ID NO:126, those amino acids of the HPV strain 16 E7 protein that would cause the polypeptide to bind to the Rb protein are excluded. Furthermore, an inserted spacer sequence identical to that present in SEQ ID NO:126 lies between the segments corresponding to residues 7-23 and 44-57, respectively.

Any of the hybrid polypeptides described herein may optionally include an initiator methionine, a leader sequence, or any targeting sequence (e.g., a transmembrane domain). The terms "targeting sequence" and "trafficking sequence" are used interchangeably herein. For example, when the third hybrid polypeptide has a methionine added to its amino terminus, it has the amino acid sequence of SEQ ID NO:158 and is encoded by a nucleotide sequence of SEQ ID NO:161. Furthermore, when the third hybrid polypeptide has an HLA-DRα leader sequence added to its amino terminus, it has the amino acid sequence of SEQ ID NO:160 and is encoded by a nucleic acid having the nucleotide sequence of SEQ ID NO:162.

Table 3 lists other MHC-binding epitopes from the HPV strain 16 E6 and E7 and the HPV strain 18 E6 and E7 polypeptides. Any of these epitopes can be incorporated into a hybrid polyepitope of the invention.

**TABLE 3: HPV E6 and E7 MHC-binding epitopes**

| **HPV 16 E6 Peptides** | **SEQ ID NO** |
|---|---|
| | |
| **A1 - binding** | |
| ISEYRHYCY | 70 |
| FQDPQERPR | 71 |
| RREVYDFAF | 72 |
| TTLEQQYNK | 73 |
| FQDPQERPRK | 74 |
| ISEYRHYCYS | 75 |
| KISEYRHYCY | 76 |
| GTTLEQQYNK | 77 |
| | |
| **A2 - binding** | |
| KLPQLCTEL | 55 |
| KISEYRHYC | 78 |
| FAFRDLCIV | 57 |
| YCYSIYGTTL | 79 |
| SEYRHYCYSL | 80 |
| | |
| **A3 - binding** | |
| AMFQDPQER | 81 |
| LLRREVYDF | 82 |
| TTLEQQYNK | 73 |
| IVYRDGNPY | 83 |
| KLPQLCTEL | 55 |
| | |
| **A11 - binding** | |
| TTLEQQYNK | 73 |
| GTTLEQQYNK | 77 |
| | |
| **A24 - binding** | |
| VYDFAFRDL | 84 |
| CYSLYGTTL | 85 |
| EYRHYCYSL | 86 |
| KLPQLCTEL | 55 |
| DPQERPRKL | 87 |
| HYCYSLYGT | 88 |
| DFAFRDLCI | 89 |
| LYGTTLEQQY | 90 |
| HYCYSLYGTT | 91 |
| EVYDFAFRDL | 92 |
| EYRHYCYSLY | 93 |
| VYDFAFRDLC | 94 |
| YCYSIYGTTL | 79 |

| **HPV 16 E7 Peptides** | **SEQ ID NO** |
|---|---|
| | |
| **A1 - binding** | |
| QAEPDRAHY | 95 |
| IVCPICSQK | 96 |
| QPETTDLY | 97 |
| QAEPDRAHYN | 98 |
| DLQPETTDLY | 99 |
| | |
| **A2 - binding** | |
| YMLDLQPET | 58 |
| TLHEYMLDL | 59 |
| LLMGTLGIV | 60 |
| LMGTLGIVC | 100 |
| MLDLQPETT | 101 |
| TLGIVCPIC | 102 |
| DLQPETTDL | 103 |
| GTLGIVCPI | 104 |
| YMLDLQPETT | 105 |
| LQPETTDLY | 106 |
| LLMGTLGIVC | 107 |
| | |
| **A3 - binding** | |
| TLHEYMLDL | 59 |
| IVCPICSQK | 96 |
| | |
| **A11 - binding** | |
| IVCPICSQK | 96 |
| | |
| **A24 - binding** | |
| DLQPETTDL | 103 |
| TLHEYMLDL | 59 |
| TPTLHEYML | 108 |
| RAHYNIVTF | 109 |
| GTLGIVCPI | 104 |
| EPDRAHYNI | 110 |

| **HPV 18 E6 Peptides** | **SEQ ID NO** |
|---|---|
| | |
| **A1 - binding** | |
| LTEVFEFAFK | 127 |
| | |
| **A2 - binding** | |
| KLPDLCTEL | 124 |
| GLYNLLIRC | 128 |
| SLQDIEITC | 129 |
| SLQDIEITCV | 130 |
| LQDIEITCV | 131 |
| KTVLELTEV | 132 |
| ELTEVFEFA | 133 |
| KLTNTGLYNL | 134 |
| LTNTGLYNL | 135 |
| GLYNLLIRCL | 125 |
| | |
| **A3 - binding** | |
| VLELTEVFEF | 136 |
| SVYGDTLEK | 137 |
| LLIRCLRCQK | 138 |
| | |
| **A11 - binding** | |
| CVYCKTVLEL | 139 |
| SVYGDTLEK | 137 |
| LLIRCLRCQK | 138 |
| | |
| **A24 - binding** | |
| VYCKTVLEL | 140 |
| VYGDTLEKL | 141 |
| LTNTGLYNLL | 142 |
| | |

| **HPV 18 E7 Peptides** | **SEQ ID NO** |
|---|---|
| | |
| **A1 - binding** | |
| HLEPQNEIPV | 143 |
| | |
| **A2 - binding** | |
| TLQDIVLHL | 144 |
| ATLQDIVLHL | 145 |
| QLFLNTLSFV | 146 |
| MLCMCCKCEA | 147 |
| CMCCKCEARI | 148 |
| FQQLFLNTL | 149 |
| TLSFVCPWC | 150 |
| | |
| **A3 - binding** | |
| HTMLCMCCK | 122 |
| | |
| **A11 - binding** | |
| HTMLCMCCK | 122 |
| | |
| **A24 - binding** | |
| QLFLNTLSF | 123 |
| FQQLFLNTL | 149 |
| AFQQLFLNTL | 151 |

### Nucleic acids encoding polypeptides containing multiple HPV epitopes

Nucleic acids encoding polyepitope polypeptides containing epitopes identified as described above can be generated using standard techniques, e.g., by overlapping PCR or linking of oligonucleotides. Preferably, codons are selected for inclusion in the construct to optimize production of the polyepitope polypeptides in bacterial or mammalian systems.

Different segments in the encoded polyepitope polypeptide, e.g., segments derived from different proteins, or from non-adjacent regions of the same protein, can be checked using a sequence analysis program, e.g., the BLAST program, to determine if the amino acid sequences at the junctions of the segments show inadvertent similarity to known human proteins. If homologies exist, the polypeptide can be redesigned to alter the order of the epitopes in the polypeptide to eliminate the regions of homology. If a particular epitope itself is over 75% identical to a portion of a known human protein, it is preferably not included in the polyepitope polypeptide.

The order of the segments within a given polyepitope polypeptide can correspond to the order in which the segments appear in the native protein, though some of the amino acid sequence between (i.e., at least one residue) the individual segments in the native protein may be deleted. Alternatively, the segment order may differ from that in the naturally occurring protein. The latter arrangement is preferable if a natural alignment results in a polypeptide having regions of homology to a human protein.

For HPV-derived epitopes, the nucleic acid construct preferably does not encode an epitope which contains the known retinoblastoma protein (Rb)-binding site in the HPV E7 protein. The construct can encode, e.g., a polypeptide which includes 2 to 5 segments from each of the HPV 16 E6, HPV 16 E7, HPV 18 E6, and HPV 18 E7 proteins, where each segment contains epitopes for binding to one or more of the HLA alleles HLA-A1, HLA-A2, HLA-A3, HLA-A11, and HLA-A24. This corresponds to about 12-20 epitopes per HLA allele, for about 60-100 epitopes in the polyepitope polypeptide.

Regulatory elements can be included in the construct to facilitate expression of the nucleic acid encoding the polyepitope polypeptide. These elements include sequences for enhancing expression in human or other mammalian cells, e.g., promoters, RNA stabilization sequences 5' and/or 3' to the coding sequence, introns (which can be placed at any location within or adjacent to the encoded sequence), and poly(A) addition sites, as well as an origin of replication and one or more genes encoding selectable markers enabling the constructs to replicate and be selected in prokaryotic and/or eukaryotic hosts. A T7 polymerase promoter or other type of promoter (e.g., a tissue-specific promoter such as a muscle-specific promoter, or a cell-specific promoter such as an APC-specific promoter) is optionally present at the 5' end of the coding sequence, and a sequence encoding a FLAG or other mAb determinant is optionally present directly 3' of the last epitope coding sequence. The construct may also contain other transcriptional and translational signals, such as a Kozak sequence.

The construct may in addition include a sequence encoding a targeting signal that directs the polyepitope polypeptide to a desired intracellular compartment, the targeting signal being linked to the polyepitope polypeptide. Targeting signals can direct the polyepitope polypeptide to endoplasmic reticulum (ER), the golgi, the nucleus, a lysosome, a class II peptide loading compartment, or an endosome, and include signal peptides (the amino terminal sequences which direct proteins into the ER during translation), ER retention peptides such as KDEL (SEQ ID NO:111), and lysosome-targeting peptides such as KFERQ (SEQ ID NO:112), QREFK (SEQ ID NO:113), and other pentapeptides having Q flanked on one side by four residues selected from K, R, D, E, F, I, V, and L. Also included are targeting signals that direct insertion of the polypeptide into a membrane (e.g., a transmembrane sequence). Polypeptides including a membrane insertion sequence can be constructed either with or without a cytoplasmic tail.

An example of an ER-targeting sequence is the HLA-DRα leader sequence, Met Ala Ile Ser Gly Val Pro Val Leu Gly Phe Phe Ile Ile Ala Val Leu Met Ser Ala Gln Glu Ser Trp Ala (SEQ ID NO:63). The targeting sequence may include only a portion (e.g., at least ten amino acid residues) of this specified 25 residue sequence, provided that the portion is sufficient to cause targeting of the polypeptide to the ER.

Nuclear localization sequences include nucleoplasmin- and SV40-like nuclear targeting signals, as described in Chelsky et al., Mol. Cell Biol. 9:2487, 1989; Robbins, Cell 64:615, 1991, and Dingwall et al., TIBS 16:478, 1991. Some nuclear localization sequences include
AVKRPAATKKAGQAKKK (SEQ ID NO:114),
RPAATKKAGQAKKKKLD (SEQ ID NO:115), and
AVKRPAATKKAGQAKKKLD (SEQ ID NO:116).

In some cases it is desirable to modify the amino acid sequence of the targeting signal to facilitate cleavage by a signal peptidase or other proteolytic agent. Recognition sequences for signal peptidases are described in Von Heijne, Nucleic Acids Research 14:4683, 1986. The -3, -1 rules of von Heijne can be used to select a sequence that increases the probability of successful cleavage by signal peptidase when the targeting signal is present.

The nucleic acids encoding the polyepitope polypeptide described herein may optionally encode a methionine residue at the amino terminus of the polypeptide to facilitate translation.

If desired, a spacer amino acid or spacer sequence can be inserted between each pair of segments. Alanine spacers have been used successfully to separate multiple individual epitopes encoded in a single DNA construct (Toes et al., Proc. Nat. Acad. Sci. (USA) 94:14660-65, 1997).

Epitope-containing segments derived from a single protein can appear in the order (i.e., from amino terminus to carboxy terminus) in which they appear in the protein. Alternatively, segments from a given protein can be arranged in an order other than that in which they appear in the native protein, and can be grouped together or mixed with segments from one or more other proteins. The construct can encode a single polyepitope polypeptide or multiple polyepitope polypeptides, each under the control of a different promoter, e.g., dual promoter vectors. A dual promoter vector permits two shorter polyepitope polypeptides to replace the single longer version, with no loss in the number of epitopes produced from a given vector. It also allows adding new epitopes without altering the sequence and perhaps the processing, of the first polyepitope polypeptide.

Nucleic acids encoding polyepitope polypeptides can be used in any vector that allows for expression in antigen-presenting cells (APC) of the patient. The vector is preferably a non-integrating viral vector or is a non-viral vector such as a plasmid or bacterial vector. An example of a suitable vector is the family of pcDNA mammalian expression vectors (Invitrogen), which permit direct and rapid cloning of PCR products. The vector can be modified to include additional epitopes, e.g., MHC class I HLA-A1, -2, -3, -11, and -24 restricted epitopes from the HPV E6 and E7 proteins of the HPV 16 and 18 strains.

To determine whether the polypeptide is processed and the expected epitopes are presented by HLA, an *in vitro* T cell stimulation assay can be performed using autologous PBL or EBV-transformed cells infected with a recombinant vaccinia virus that contains the polyepitope polypeptide coding sequence. These target cells are generated by incubating PBL with the recombinant vaccinia at an m.o.i of 3-10 pfu/cell at 37°C for 2 h. After infection, cells are pelleted, washed and used as targets in the *in vitro* stimulation assay. The stimulated T cells from one or more individuals with the different HLA allotypes are incubated with the target cells, and the ability of the target cells to stimulate the T cells is measured, e.g., by γ-interferon expression or secretion.

Alternatively, epitope processing from the polyepitope polypeptide can be examined using proteasomes purified from human cells (Theobald et al., J. Exp. Med. 188:1017, 1998; Kisselev et al., J. Biol. Chem. 289:3363, 1999; and Nussbaum et al., Proc. Nat. Acad. Sci. (USA) 95:12404, 1998).

In addition to the T cell assays, an assay that utilizes transgenic animals can be used to verify that the construct functions (e.g., epitopes are correctly processed and presented) when delivered *in vivo.* For measuring HLA-A2-restricted presentation, the polyepitope construct in a mammalian expression vector (e.g., a plasmid) is encapsulated in microspheres and introduced into HLA-A2 transgenic mice by a route such as intramuscular or subcutaneous injection. The construct may alternatively be administered without the microsphere delivery vehicle, e.g., in a recombinant vaccinia virus or as naked DNA. T cell responses are subsequently examined *in vitro* (Hedley et al., Nature Med. 4:365-68, 1998). Target cells are T2A2 cells (T2 cells transfected with DNA encoding HLA-A2) or EL4.A2 cells (EL4 cells transfected with DNA encoding HLA-A2) pulsed with the A2 epitope being tested and T2A2 cells into which has been introduced a nucleic acid of the invention. Parallel studies are performed using T2A2 cells pulsed with no peptide or with an irrelevant peptide. In this way, HLA-A2 epitopes that are processed and presented *in vivo* following administration of the nucleic acid of the invention are identified. A positive result suggests that processing of the polyepitope polypeptide is occurring as predicted.

The nucleic acid encoding the polyepitope polypeptide, as well as the polyepitope polypeptide itself, can be used in manufacture of a medicament for the prevention or treatment of a tumor or an infection with a pathogen (e.g., HPV), or conditions associated with such infection.

### Delivery of Epitopes and Nucleic Acids Encoding Immunogenic Epitopes

Various art-recognized delivery systems may be used to deliver polyepitope polypeptides, or nucleic acids encoding polyepitope polypeptides, into appropriate cells. An advantage of DNA delivery of antigenic MHC class I-restricted epitopes is that the epitopes are produced inside the target cell itself, where the interaction with a class I MHC molecule to which the immunogenic epitope binds is kinetically favored. This is in contrast to standard vaccine protocols which do not specifically direct antigenic epitopes to MHC molecules intracellularly so that the epitopes may bind with the MHC molecules prior to presentation of the MHC molecules on the cell surface.

The polyepitope polypeptides and nucleic acids encoding the polyepitope polypeptides can be delivered in a pharmaceutically acceptable carrier such as saline, or as colloidal suspensions, or as powders, with or without diluents. They can be "naked" or associated with delivery vehicles and delivered using delivery systems known in the art, such as lipids, liposomes, microspheres, microparticles or microcapsules, gold particles, ISCOMS, nanoparticles, polymers, condensing agents, polysaccharides, polyamino acids, dendrimers, saponins, QS21, adsorption enhancing materials, adjuvants, or fatty acids. Examples of suitable microparticles are presented below.

The polyepitope polypeptides, or nucleic acids encoding the polyepitope polypeptides, can be administered using standard methods, e.g., those described in Donnelly et al., J. Imm. Methods 176:145, 1994, and Vitiello et al., J. Clin. Invest. 95:341, 1995, and can be delivered into subjects in any mariner known in the art, e.g., orally intramuscularly, intravenously, intraarterially, intrathecally, intradermally, intraperitoneally, intranasally, intrapulmonarily, intraocularly, intravaginally, intrarectally or subcutaneously. They can be introduced into the gastrointestinal tract or the respiratory tract, e.g., by inhalation of a solution or powder containing the microparticles. Administration can be local (e.g., at the cervix, skin, or other site of HPV infection) or systemic.

It is expected that a dosage of approximately 0.1 to 100 µmoles of the polypeptide, or of about 1 to 2000 µg of DNA, would be administered per kg of body weight per dose. Where the patient is an adult human, vaccination regimens can include, e.g., intramuscular, intravenous, oral, or subcutaneous administrations of 10-1000 µg of a plasmid DNA when delivered in a microparticle, or of about 10-2500 µg, e.g., 100 to 2000, or 500 to 1000 µg, of naked plasmid DNA delivered intramuscularly or intradermally, repeated 3-6 times. Of course, as is well known in the medical arts, dosage for any given patient depends upon many factors, including the patient's size, general health, sex, body surface area, age, the particular compound to be administered, time and route of administration, and other drugs being administered concurrently. Determination of optimal dosage is well within the abilities of a pharmacologist of ordinary skill.

Other standard delivery methods, e.g., biolistic transfer or *ex vivo* treatment, can also be used. In *ex vivo* treatment, antigen presenting cells (APCs) such as dendritic cells, peripheral blood mononuclear cells, or bone marrow cells can be obtained from a patient or an appropriate donor and activated *ex vivo* with the immunogenic compositions, and then implanted or reinfused into the patient.

The nucleic acids encoding the polyepitope polypeptides, or the polyepitope polypeptides themselves, can be administered alone or in combination with other therapies known in the art, e.g., chemotherapeutic regimens, radiation, and surgery, to treat tumors or infection, e.g., HPV infections or diseases associated with HPV infections In addition, the polypeptides and nucleic acids of the invention can be administered in combination with other treatments designed to enhance immune responses, e.g., by co-administration with adjuvants or cytokines (or nucleic acids encoding cytokines), as is well known in the art.

### Microsphere Delivery

In one preferred delivery method, nucleic acids encoding the polyepitope polypeptides, or the polyepitope polypeptides themselves, are delivered using microspheres. Microspheres, including those described in U.S. Patent No. 5,783,567, can be used as vehicles for delivering macromolecules such as DNA, RNA, or polypeptides into cells. The microspheres contain the macromolecules embedded in a polymeric matrix or enclosed in a hollow shell of polymer. Solid microspheres may also be formed for example as in WO 95/24929, herein incorporated by reference. The term microsphere, as used herein, includes microparticles and microcapsules. Microspheres act to maintain the integrity of the macromolecule, e.g., by maintaining the encapsulated DNA in a nondegraded state. Microspheres of an appropriate size or combination of sizes can also be used for pulsed delivery of the macromolecule, and for delivery at a specific site or to a specific target cell population.

The polymeric matrix can be a biodegradable polymer such as polylactide-co-glycolide(PLG), polylactide, polyglycolide, polyanhydride, polyorthoester, polycaprolactone, polyphosphazene, proteinaceous polymer, polypeptide, polyester, or a naturally occurring polymer such as starch, alginate, chitosan, and gelatin.

The microspheres can also include one or more stabilizer compounds (e.g., a carbohydrate, a cationic compound, a pluronic, e.g., Pluronic-F68^{™} (Sigma-Aldrich Co., St. Louis, MO), a lipid, or a DNA-condensing agent). A stabilizer compound is a compound that acts to protect the nucleic acid (e.g., to keep it supercoiled or protect it from degradation) at any time during the production of microspheres or after *in vivo* delivery. The stabilizer compound can remain associated with the DNA after a later release from the polymeric delivery system.

Examples of stabilizer compounds include tris(hydroxymethyl)aminomethane (TRIS), ethylenediaminetetraacetic acid (EDTA), or a combination of TRIS and EDTA (TE). Other stabilizer compounds include dextrose, sucrose, lactose, dextran, trehalose, cyclodextrin, dextran sulfate, cationic peptides, pluronics, e.g., Pluronic F-68^{™} (Sigma-Aldrich Co., St. Louis, MO), and lipids such as hexadecyltrimethylammonium bromide. Preparation of microspheres containing stabilizer agents is described in USSN 09/266,463.

The lipid can be a charged lipid such as a cationic lipid, an anionic lipid (such as PEG-DSPE, taurocholic acid or phosphatidyl inositol), or a zwitterionic lipid, or may have no charge. Examples of lipids include cetyltrimethylammonium and phospholipids, e.g., phosphatidylcholine. The microspheres may contain one or more than one type of lipid, e.g., those lipids present in lecithin lipid preparations, and may also include one or more stabilizer compounds as described above.

Microspheres can be used to maximize delivery of DNA molecules into a subject's phagocytotic cells. Alternatively, the biodegradable microspheres can be injected or implanted in a tissue, where they form a deposit. As the deposit breaks down, the nucleic acid is released gradually over time and taken up by neighboring cells (including APCs) as free DNA.

### Delivery Using Other Agents

The polyepitope polypeptides, or nucleic acids encoding them, can also be administered to subjects using other agents such as lipids, dendrimers, or liposomes, using techniques that are well known in the art. For example, liposomes carrying either immunogenic polypeptides or nucleic acids encoding immunogenic epitopes are known to elicit CTL responses *in vivo* (Reddy et al., J. Immunol. 148:1585, 1992; Collins et al., J. Immunol. 148:3336-3341, 1992; Fries et al., Proc. Natl. Acad. Sci. (USA) 89:358, 1992; Nabel et al., Proc. Natl. Acad. Sci. (USA) 89:5157, 1992).

The polypeptides and nucleic acids of the invention can also be administered by using Immune Stimulating Complexes (ISCOMS), which are negatively charged, cage-like structures 30-40nm in size formed spontaneously on mixing cholesterol and Quil A (saponin), or from saponin alone. The polypeptides and nucleic acids of the invention can be complexed with ISCOMs, then administered, or can be administered separately.

Protective immunity has been generated in a variety of experimental models of infection, including toxoplasmosis and Epstein-Barr virus-induced tumors, using ISCOMs as the delivery vehicle for antigens (Mowat et al., Immunology Today 12:383-385, 1991). Doses of antigen as low as 1µg encapsulated in ISCOMs have been found to produce class I- mediated CTL responses, where either purified intact HIV-1-IIIB gp 160 envelope glycoprotein or influenza hemagglutinin is the antigen (Takahashi et al., Nature 344:873-875, 1990).

### Measuring Immune Responses

The ability of polyepitope polypeptides, or nucleic acids encoding them, to elicit an immune response in a host mammal can be assayed by using methods for measuring immune responses that are well known in the art. For example, the generation of cytotoxic T cells can be demonstrated in a standard ⁵¹Cr release assay, by measuring intracellular cytokine expression or secretion, or by using MHC tetramers. Standard assays, such as ELISA or ELISPOT, can be used to measure cytokine profiles attributable to T cell activation. T cell proliferation can be measured using assays such as ³H-thymidine uptake and other assays known in the art. B cell responses can be measured using art recognized assays such as ELISA.

Other methodologies, e.g., digital imaging and cytologic, colposcopic and histological evaluations, can also be used to evaluate the effects of immunogenic epitopes, and of nucleic acids encoding the immunogenic epitopes, on pathogen-associated lesions, or on viral or other pathogen levels generally.

Particular embodiments of the invention are:
1. A nucleic acid encoding a hybrid polypeptide comprising a signal sequence and three segments, wherein the three segments are either contiguous or are separated by a spacer amino acid or spacer peptide:
   (a) the first segment having the amino acid sequence of a first portion of a naturally occurring tumor antigen or naturally occurring protein of a pathogenic agent, the first segment being at least eleven amino acids in length and comprising two epitopes;
   (b) the second segment having the amino acid sequence of a second portion of a naturally occurring tumor antigen or naturally occurring protein of a pathogenic agent, the second segment being at least eleven amino acids in length and comprising two epitopes different from the epitopes of (a); and
   (c) the third segment having the amino acid sequence of a third portion of a naturally occurring tumor antigen or naturally occurring protein of a pathogenic agent, the third segment being at least eleven amino acids in length and comprising two epitopes different from the epitopes of (a) and (b), provided that either
      (i) the first, second and third portions are non-contiguous portions of the same naturally occurring protein, and the sum of all three portions constitutes less than 70% of the sequence of the naturally occurring protein; or
      (ii) the first, second and third portions are portions of three different naturally occurring tumor antigens or naturally occurring proteins of one or more pathogenic agents.
2. The nucleic acid of embodiment 1, wherein at least one of the segments comprises three epitopes.
3. The nucleic acid of embodiment 1, wherein at least one of the segments comprises four epitopes.
4. The nucleic acid of embodiment 1, wherein at least three of the epitopes are MHC class I-binding epitopes.
5. The nucleic acid of embodiment 1, further comprising (d) a fourth segment which has the amino acid sequence of a fourth portion of a naturally occurring tumor antigen or naturally occurring protein of a pathogenic agent, the fourth segment being at least eleven amino acids in length and comprising two epitopes different from the epitopes of (a), (b) and (c).
6. The nucleic acid of embodiment 5, wherein the fourth segment has the amino acid sequence of a portion of a naturally occurring protein that is different from the naturally occurring protein of (a).
7. The nucleic acid of embodiment 1, wherein at least one of the segments is less than 15 amino acids in length.
8. The nucleic acid of embodiment 1, wherein at least one of the segments has the sequence of a portion of a human papilloma virus (HPV) protein.
9. The nucleic acid of embodiment 1, wherein each of the naturally occurring proteins is an HPV protein.
10. The nucleic acid of embodiment 1, wherein at least two of the segments are contiguous.
11. The nucleic acid of embodiment 1, wherein the three segments are contiguous.
12. The nucleic acid of embodiment 1, wherein the first and second segments are separated by a spacer amino acid or a spacer peptide and the second and third segments are separated by a spacer amino acid or a spacer peptide.
13. The nucleic acid of embodiment 1, wherein the first and second segments are separated by a spacer amino acid and the second and third segments are separated by a spacer amino acid.
14. The nucleic acid of embodiment 1, wherein the first and second segments are separated by a spacer amino acid which is alanine and the second and third segments are separated by a spacer amino acid which is alanine.
15. The nucleic acid of embodiment 12, wherein each of the naturally occurring proteins is an HPV protein.
16. The nucleic acid of embodiment 13, wherein each of the naturally occurring proteins is an HPV protein.
17. The nucleic acid of embodiment 14, wherein each of the naturally occurring proteins is an HPV protein.
18. The nucleic acid of embodiment 8, wherein the hybrid polypeptide comprises a first epitope from an HPV protein and a second epitope which does not overlap with the first epitope and which is from the same or a different HPV protein,
   wherein the first epitope binds to a first major histocompatibility complex (MHC) class I allotype and the second epitope binds to a second MHC class I allotype different from the first MHC class I allotype.
19. The nucleic acid of embodiment 18, wherein at least one of the portions is from an HPV E6 or HPV E7 protein.
20. The nucleic acid of embodiment 18, wherein at least one of the portions is from an HPV strain 16 protein or an HPV strain 18 protein.
21. The nucleic acid of embodiment 18, wherein at least one of the portions is from an HPV E6 or E7 protein of HPV strain 16 or 18 origin.
22. The nucleic acid of embodiment 18, wherein the first MHC class I allotype is selected from the group consisting of HLA-A1, HLA-A2, HLA-A3, HLA-A11, and HLA-A24.
23. The nucleic acid of embodiment 22, wherein the second MHC class I allotype is selected from the group consisting of HLA-A1, HLA-A2, HLA-A3, HLA-A11, and HLA-A24.
24. The nucleic acid of embodiment 18, wherein the hybrid polypeptide further comprises a third epitope from an HPV protein, wherein the third epitope binds to a third MHC class I allotype different from the first and second MHC class I allotypes.
25. The nucleic acid of embodiment 18, wherein the hybrid polypeptide comprises 10 MHC class I allotype-binding epitopes from one or more HPV proteins.
26. The nucleic acid of embodiment 18, wherein the hybrid polypeptide comprises 40 MHC class I allotype-binding epitopes from one or more HPV proteins.
27. The nucleic acid of embodiment 18, wherein the hybrid polypeptide comprises 60 MHC class I allotype-binding epitopes from one or more HPV proteins.
28. The nucleic acid of embodiment 24, wherein the first epitope overlaps with the third epitope.
29. The nucleic acid of embodiment 1, wherein the signal sequence and the first segment are separated by a spacer amino acid or a spacer peptide.
30. The nucleic acid of embodiment 1, wherein the hybrid polypeptide comprises ten MHC class I-binding epitopes from one HPV protein.
31. The nucleic acid of embodiment 1, comprising
   (a) a first plurality of HLA-binding epitopes from an HPV strain 16 E6 protein, and
   (b) a second plurality of HLA-binding epitopes from an HPV strain 16 E7 protein;
   wherein each of the HLA-binding epitopes binds to one or more allotypes selected from the group consisting of HLA-A1, HLA-A2, HLA-A3, HLA-A11, and HLA-A24.
32. The nucleic acid of embodiment 1, comprising
   (a) a first plurality of HLA-binding epitopes from an HPV strain 18 E6 protein, and
   (b) a second plurality of HLA-binding epitopes from an HPV strain 18 E7 protein,
   wherein each of the HLA-binding epitopes binds to one or more allotypes selected from the group consisting of HLA-A1, HLA-A2, HLA-A3, HLA-A11, and HLA-A24.
33. The nucleic acid of embodiment 1, comprising
   (a) a first plurality of HLA-binding epitopes from an HPV strain 16 E6 or E7 protein, and
   (b) a second plurality of HLA-binding epitopes from an HPV strain 18 E6 or E7 protein,
   wherein each of the HLA-binding epitopes binds to one or more allotypes selected from the group consisting of HLA-A1, HLA-A2, HLA-A3, HLA-A11, and HLA-A24.
34. The nucleic acid of embodiment 1, comprising
   (a) a first plurality of HLA-binding epitopes from an HPV strain 16 E6 protein,
   (b) a second plurality of HLA-binding epitopes from an HPV strain 16 E7 protein,
   (c) a third plurality of HLA-binding epitopes from an HPV strain 18 E6 protein, and
   (b) a fourth plurality of HLA-binding epitopes from an HPV strain 18 E7 protein,
      wherein each of the HLA-binding epitopes binds to one or more allotypes selected from the group consisting of HLA-A1, HLA-A2, HLA-A3, HLA-A11, and HLA-A24.
35. The nucleic acid of embodiment 31, wherein each plurality of epitopes comprises at least five epitopes, each of which binds to one or more of the allotypes.
36. The nucleic acid of embodiment 31, wherein each plurality of epitopes comprises at least 15 epitopes, each of which binds to one or more of the allotypes.
37. A nucleic acid encoding a hybrid polypeptide comprising a signal sequence and three segments, wherein the three segments are either contiguous or are separated by a spacer amino acid or spacer peptide:
   (a) the first segment having the amino acid sequence of a first portion of a naturally occurring HPV protein, the first segment being at least eleven amino acids in length and comprising two epitopes;
   (b) the second segment having the amino acid sequence of a second portion of a naturally occurring HPV protein, the second segment being at least eleven amino acids in length and comprising two epitopes different from the epitopes of (a); and
   (c) the third segment having the amino acid sequence of a third portion of a naturally occurring HPV protein, the third segment being at least eleven amino acids in length and comprising two epitopes different from the epitopes of (a) and (b), provided that either

   (i) the first, second and third portions are non-contiguous portions of the same naturally occurring HPV protein, and the sum of all three portions constitutes less than 70% of the sequence of the naturally occurring protein; or
   (ii) the first, second and third portions are portions of two or three different naturally occurring HPV proteins.
38. The nucleic acid of embodiment 37, wherein at least one of the segments comprises three epitopes.
39. The nucleic acid of embodiment 37, wherein at least one of the segments comprises five epitopes.
40. The nucleic acid of embodiment 37, wherein at least three of the epitopes are MHC class I-binding epitopes.
41. The nucleic acid of embodiment 37, further comprising (d) a fourth segment which has the amino acid sequence of a fourth portion of a naturally occurring HPV protein, the fourth segment being at least eleven amino acids in length and comprising two epitopes different from the epitopes of (a), (b) and (c).
42. A DNA encoding a hybrid polypeptide the sequence of which comprises at least one of the following segments of HPV strain 16 E6:
   AMFQDPQERPRKLPQLCTEL (SEQ ID NO:64),
   LLRREVYDFAFRDLCIVYRDGNPY (SEQ ID NO:65), and
   KISEYRHYCYSLYGTTLEQQYNK (SEQ ID NO:66), and at least one of the following segments of HPV strain 16 E7:
   TLHEYMLDLQPETTDLYSY (SEQ ID NO:67),
   QAEPDRAHYNIVTF (SEQ ID NO:68), and
   LLMGTLGIVCPICSQKP (SEQ ID NO:69), provided that the hybrid polypeptide does not comprise a sequence identical to the sequence of either full length, intact E6 or full length, intact E7 protein from HPV strain 16.
43. The DNA of embodiment 42, wherein the hybrid polypeptide comprises at least three of the segments.
44. The DNA of embodiment 42, wherein the hybrid polypeptide comprises all six of the segments.
45. A DNA encoding a hybrid polypeptide the sequence of which comprises at least one of the following segments of HPV strain 16 E6 and E7 proteins:
   AMFQDPQERPRKLPQLCTEL (SEQ ID NO:64),
   LLRREVYDFAFRDLCIVYRDGNPY (SEQ ID NO:65),
   KISEYRHYCYSLYGTTLEQQYNK (SEQ ID NO:66),
   TLHEYMLDLQPETTDLYSY (SEQ ID NO:67), and
   QAEPDRAHYNIVTF (SEQ ID NO:68), provided that the hybrid polypeptide does not comprise a sequence identical to the sequence of either full length, intact E6 or full length, intact E7 protein from HPV strain 16.
46. A DNA encoding a hybrid polypeptide the sequence of which comprises at least one of the following segments of HPV strain 18 E6:
   RRPYKLPDLCTELNTSLQDIEITCVYCKTVLELTEVFEFAFK (SEQ ID NO:152), and
   SVYGDTLEKLTNTGLYNLLIRCLRCQK (SEQ ID NO:153), and at least one of the following segments of HPV strain 18 E7:
   KATLQDIVLHLEPQNEIPV (SEQ ID NO:154),
   HTMLCMCCKCEARI (SEQ ID NO:155), and
   AFQQLFLNTLSFVCPWC (SEQ ID NO:156), provided that the hybrid polypeptide does not comprise a sequence identical to the sequence of either full length, intact E6 or full length, intact E7 protein from HPV strain 18.
47. A DNA encoding a hybrid polypeptide the sequence of which comprises at least one of the following segments of HPV strain 16 E6:
   AMFQDPQERPRKLPQLCTEL (SEQ ID NO:64),
   LLRREVYDFAFRDLCIVYRDGNPY (SEQ ID NO:65), and
   KISEYRHYCYSLYGTTLEQQYNK (SEQ ID NO:66); at least one of the following segments of HPV strain 16 E7:
   TLHEYMLDLQPETTDLYSY (SEQ ID NO:67),
   QAEPDRAHYNIVTF (SEQ ID NO:68), and
   LLMGTLGIVCPICSQKP (SEQ ID NO:69); at least one of the following segments of HPV strain 18 E6:
   RRPYKLPDLCTELNTSLQDIEITCVYCKTVLELTEVFEFAFK (SEQ ID NO:152), and
   SVYGDTLEKLTNTGLYNLLIRCLRCQK (SEQ ID NO:153), and at least one of the following segments of HPV strain 18 E7:
   KATLQDIVLHLEPQNEIPV (SEQ ID NO:154),
   HTMLCMCCKCEARI (SEQ ID NO:155), and
   AFQQLFLNTLSFVCPWC (SEQ ID NO:156).
48. The DNA of embodiment 47, wherein the hybrid polypeptide comprises at least five of the segments.
49. The DNA of embodiment 47, wherein the hybrid polypeptide comprises all eleven of the segments.
50. The DNA of embodiment 49, wherein the hybrid polypeptide further comprises a targeting signal.
51. The DNA of embodiment 50, wherein the targeting signal comprises the HLA-DRα leader sequence (SEQ ID NO:63).
52. A DNA encoding a hybrid polypeptide the sequence of which comprises at least one of the following segments of HPV E6 and E7 proteins:
   AMFQDPQERPRKLPQLCTEL (SEQ ID NO:64),
   LLRREVYDFAFRDLCIVYRDGNPY (SEQ ID NO:65),
   KISEYRHYCYSLYGTTLEQQYNK (SEQ ID NO:66),
   TLHEYMLDLQPETTDLYSY (SEQ ID NO:67),
   QAEPDRAHYNIVTF (SEQ ID NO:68),
   RRPYKLPDLCTELNTSLQDIEITCVYCKTVLELTEVFEFAFK (SEQ ID NO:152),
   SVYGDTLEKLTNTGLYNLLIRCLRCQK (SEQ ID NO:153),
   KATLQDIVLHLEPQNEIPV (SEQ ID NO:154),
   HTMLCMCCKCEARI (SEQ ID NO:155), and
   AFQQLFLNTLSFVCPWC (SEQ ID NO:156); provided that the hybrid polypeptide does not comprise a sequence identical to the sequence of either full length, intact E6 or full length, intact E7 protein from HPV strain 16 or 18.
53. A plasmid or viral vector comprising the nucleic acid of embodiment 1.
54. The hybrid polypeptide encoded by the nucleic acid of embodiment 1.
55. A microsphere comprising a polymeric matrix or shell and the nucleic acid of embodiment 1.
56. The microsphere of embodiment 55, wherein the polymeric matrix or shell consists essentially of a polymer of poly-co-glycolic acid (PLGA).
57. A therapeutic composition comprising the nucleic acid of embodiment 1 and a pharmaceutically acceptable carrier.
58. The therapeutic composition of embodiment 57, further including an adjuvant.
59. A liposome comprising the nucleic acid of embodiment 1.
60. A method of eliciting an immune response in a mammal, which method comprises administering the nucleic acid of embodiment 1 to the mammal.
61. The method of embodiment 60, wherein the mammal is a human.
62. The method of embodiment 61, wherein the pathogenic agent is HPV and the human suffers from, or is at risk of, a condition selected from the group consisting of exophytic condyloma, flat condyloma, cervical cancer, respiratory papilloma, conjunctival papilloma, genital-tract HPV infection, cervical dysplasia, high grade squamous intraepithelial lesions, and anal HPV infection.
63. The method of embodiment 60, wherein the nucleic acid is administered directly to a mucosal tissue of the mammal.
64. The method of embodiment 63, wherein the mucosal tissue is vaginal or anal tissue.
65. The method of embodiment 60, wherein the nucleic acid is administered subcutaneously or intramuscularly.
66. A method of eliciting an immune response in a mammal, which method comprises administering the microsphere of embodiment 55 to the mammal.
67. The nucleic acid of embodiment 1, wherein the first, second and third portions are portions of one or more tumor antigens expressed from a gene selected from the group consisting of the Her2/neu gene, the prostate specific antigen (PSA) gene, the melanoma antigen recognized by T cells (MART) gene, and the melanoma antigen gene (MAGE).
68. The nucleic acid of embodiment 1, wherein the first, second and third portions are portions of one or more naturally occurring proteins of one or more viruses which infect cells.
69. The nucleic acid of embodiment 1, wherein the first, second and third portions are portions of one or more naturally occurring proteins of one or more pathogenic agents selected from the group consisting of HPV, human immunodeficiency virus (HIV), herpes simplex virus (HSV), hepatitis B virus (HBV), hepatitis C virus (HCV), mycobacteria, *Helicobacter spp., Chlamydia* spp., and a parasitic eukaryote which infects cells.

The following are examples of the practice of the invention. They are not to be construed as limiting the scope of the invention in any way.

### Example 1. Identification of HPV-derived MHC class I-binding epitopes

HLA alleles are isolated from cell lines which express a single HLA-A allotype. 10-20 liters of each cell line (about 1-2 H 10¹⁰ cells) are grown in complete RPMI-1640 media (10% FCS, HEPES, Pen/Strep, essential amino acids, glutamine) in roller bottles, and the cells harvested by centrifugation (10-15 gm wet weight cells/10 L culture). A membrane preparation is generated by lysing cells in 10 mM Tris, 1 mM DTT, 0.1 mM PMSF, for 30 min. Debris is pelleted, after which the cleared lysate is homogenized in lysis buffer and again pelleted. The pellet is homogenized in buffer containing 4% NP-40 and ultracentrifuged. The detergent-soluble material is used for HLA purification.

The solubilized membrane preparation is pumped through pre-clearing columns (chromatographic matrix and normal mouse serum-matrix) before the protein/ligand containing effluent is directed towards one, or a series of, specific immunoaffinity column(s). Immunoaffinity columns containing the pan anti-class I mAb W6/32 can be used to isolate class I molecules from the single allotype-expressing cell lines. Alternatively, allotype-specific mAb such as BB7.2 are used to extract a single HLA allotype (HLA-A2) from a cell lysate that expresses multiple allotypes. Coupling of mAb to high strength, large throughpore perfusion sorbents (coated and crosslinked with a hydrophilic stationary phase and covalently attached to Protein A) can be utilized to allow for fast flowrates (up to 20 ml/min). The immunoaffinity columns are then extensively washed and the protein/ligand complex is eluted from the immunoaffinity support using 50 mM carbonate/0.1% DOC/0.05% NaN₃ at pH 11.5.

Multi-modal high-performance liquid-chromatography (HPLC) separation of HLA molecules is achieved by coupling the chromatographic procedures in series with automated switching valves, which direct the protein/ligand-containing effluent to subsequent columns in the sequence. Each column effluent can be monitored at multiple UV wavelengths, pressure, and pH.

Purified HLA alleles are then used in epitope binding competition assays.
Binding by a putative epitope is compared to binding by previously described HLA-binding epitopes. Examples of known epitopes and their respective allotypes include: HLA-A1, YLEPAIAKY (SEQ ID NO:117); HLA-A2, FLPSDYFPSV (SEQ ID NO:118); HLA-A3, KVFPYALINK (SEQ ID NO:119); HLA-A11, AVDLYHFLK (SEQ ID NO:120); and HLA-A24, AYIDNYNKF (SEQ ID NO:121). General assay conditions are as follows: class I proteins are incubated in 0.05% NP-40/PBS with ∼5 nM of radiolabeled standard epitope and the test inhibitor epitope in presence of 1 µM human β2microglobulin, and a mixture of protease inhibitors (final concentration 1 mM PMSF, 1.3 mM 1,10 phenanthroline, 73 µM pepstatin, 8 mM EDTA and 200 µM N-α-p-tosyl-L-lysine chloromethyl ketone) at 23EC for 48 h. The final concentration of each HLA allotype is determined experimentally as explained below. Concentrations of inhibitor peptides are titrated at concentration ranges from 1 nM to 100 µM.

Free and bound peptides are separated by HPLC size exclusion chromatography using a TSK 2000^{™} SEC column and 0.5% NP-40/PBS. The eluent is monitored for radioactivity and the fraction of peptide bound to HLA relative to the total amount of offered peptide is calculated from the ratio of peptide in the void volume to the total peptide recovered. The final concentration of HLA-A1, -A2, -A3, -A11, and BA24 to be used in subsequent binding assays must be determined experimentally based on the binding efficiency for the radiolabeled standard peptide. The necessary protein concentration for inhibition studies typically falls between 10-40 nM. Each allotype is tested for binding of the radiolabeled peptide by performing serial dilutions of the protein concentration from 1 nM to 1 µM of the HLA molecule at the beginning of the screening.

The binding affinity of potential peptide epitopes to human leukocyte antigen (HLA) receptors can be studied using a competitive binding assay (Sette et al., Molecular Immunology 31:813-822, 1994). This assay requires (1) purified HLA receptors, (2) a synthetic peptide which binds to the HLA receptor of interest and contains a tyrosine amino acid in a location which can be labeled with radioactive iodine-125 without interrupting the peptide's ability to bind, (3) purified beta-2-microglobulin (β₂m), and (4) synthetic peptide epitopes which are to be tested. The optimal experimental HLA receptor concentrations are established by titrating the amount of HLA receptor in each binding assay required to achieve at least 10% binding of the total labeled peptide (fixed at 5nM) (β₂m concentration is also fixed at 1 µM). With these three parameters (concentration of receptor, β₂m, and concentration of radiolabeled peptide) held constant, a titration of unlabeled test peptide is performed. Each binding reaction is incubated at room temperature for 30-80 h to allow for peptide exchange. The quantitation of the peptide exchange is determined by separating the bound fraction of radiolabeled peptide/receptor complex away from the excess free peptide and β₂m by size exclusion chromatography. Using an HPLC system fitted with a radioisotope detector, the percentage of radiolabeled peptide bound to the HLA receptor can be quantified. The ability of the unlabeled test peptide to inhibit the binding of the labeled peptide is then plotted as a function of its concentration. The affinity of the test peptide is presented as the concentration of test peptide required to inhibit 50% of the total binding of the radiolabeled peptide (a so-called IC-50 value).

Several peptides derived from the E6 and E7 proteins of the human papilloma virus (HPV) strains 16 and 18 have been identified as high affinity, HLA-binding peptides using this type of assay. In these experiments, HLA receptors A1 and A11 were purified from human cells which had been transfected with HLA-A*0101 and HLA-A*1101 genes, respectively (Gorga et al., J. Biol. Chem. 262:16087-16094, 1987). In the case of HLA receptors A3 and A24, recombinant HLA receptors (HLA-A*0301 and HLA-A*2402) were used which had been produced in E. coli and refolded (Garboczi et al., Proc. Natl. Acad. Sci. USA 89:3429-3433, 1992). Examples of experimental conditions and data collected are presented in Table 4.

**TABLE 4**

| Sequence (amino acid) | HLA Type | Incubation (hours) | IC-50 (nM) |
|---|---|---|---|
| ISEYRHYCY (SEQ ID NO:70) | A1 | 51 | 49 |
| IVYRDGNPY (SEQ ID NO:83) | A3 | 46 | 238 |
| HTMLCMCCK (SEQ ID NO:122) | A11 | 68 | 67 |
| QLFLNTLSF (SEQ ID NO:123) | A24 | 68 | 47 |
| SLQDIEITCV (SEQ ID NO:130) | A2 | 50.5 | 50 |

If desired, the predictive binding algorithm described above can be used to prioritize putative epitopes according to dissociation times for each allele, with epitopes having the longest dissociation time tested first. After 5-10 epitopes with reasonable affinities for each of the dominant HLA-A alleles (i.e., < 500nM) are identified, testing can be discontinued. If epitopes with less than 500nM binding affinity are not found, then the 5-10 best binders from each protein can be selected for continued analysis.

Peptides can also be identified, for example, according to the methods of Parker, et al. (J. Immunol. 149: 1896-1904, 1992; J. Immunol. 149: 2580-3587, 1992; J. Biol. Chem. 267:5451-5459, 1992) and Garboczi, et al. (PNAS 89:3429-3433, 1992), herein incorporated by reference.

### Example 2. Detection of MHC class I-restricted epitope presentation in vitro

Peripheral blood mononuclear cells (PBMC) from normal volunteers are purified by Ficoll-Paque^{™} (Pharmacia, Piscataway, NJ) density centrifugation from leukophoresis products screened as HIV, HBV, and HCV seronegative. Dendritic cells (DC) are generated in tissue culture from monocyte-enriched PBMC fractions as described (Tsai et al., Crit. Rev. Immunol. 18:65-75, 1998; Wilson et al., J. Immunol. 162:3070-78, 1999). 10⁷ PBMC/ml in serum-free RPMI 1640 medium (Gibco-BRL) are plated in flasks and incubated 1.5-2.0 hr at 37EC. Non-adherent cells are removed by gentle washes, and the plastic-adherent monocytes containing DC precursors are cultured in complete medium in the presence of 50 ng/ml GM-CSF and 1000 U/ml IL-4 (both from R&D Systems, Minneapolis, MN) for 6-7 days. Complete medium consists of RPMI 1640 supplemented with 5% pooled human AB serum (C-6 Diagnostics, Mequon, WI), L-glutamine, penicillin/streptomycin, 2-ME (Gibco-BRL, Gaithersburg, MD) and HEPES (JRH Biosciences, Lenexa, KS) at the recommended concentrations.

Non-adherent cells are collected by centrifugation and prepared directly for use as APC or cryopreserved. Cells are determined to be DC by morphology and by expression of a CD3/CD16-negative, MHC class I^{hi}, MHC class II^{hi}, CD86-positive phenotype as assessed by cytofluorimetry.

At Day 0, 5x10⁵ non-adherent PBL are plated with 2.5x10⁴ irradiated, epitope-pulsed DC. The DC are in 48 well plates in 0.5 ml complete RPMI-1640 with 10% normal human AB serum supplemented with 10 ng/ml IL-7. The DC are incubated with the PBL at 37°C in a CO₂ incubator. 10 ng/ml IL-10 is added 24 h later. On day 7, autologous PBL are thawed and irradiated. 1x10⁶ cells per well are plated in a 48 well plate and allowed to adhere for 2 h. Epitopes are added, and cells are pulsed for 2 h. Cells are washed once, and responders transferred into wells with adherent stimulators. 10 ng/ml IL-10 is added 24 h later. 20 U IL-2/ml are added on day 9 and 100 U/ml IL-2 on day 11. Cells are restimulated on day 15 in the same fashion. On day 22, the cells in 1-2 wells of each individual culture are harvested and counted. Responders are those cells that have increased cell number 1.5-3 fold over day 16 input. The total culture yields generally should be at least 2x10⁶ cells.

Cells to be used as targets are harvested and adjusted to 6.67x10⁵ cells/ml in medium. Two mls of cells are pulsed with 50 ug/ml of each of the appropriate epitopes (e.g., Flu M1, Test #1, Test #2, Test #3, Test #4). Effectors are harvested and resuspended at 1x10⁶ cells/ml in medium. 100 µl is plated into each well of a round bottom 96-well plate. 150 µl of each respective target cell is aliquotted to the well containing the appropriate effector cells for a total volume of 250 µl/well. Plates are incubated for 24 h in an humidified 37°C CO₂ incubator.

ENDOGEN (Woburn, MA) human IFNγ ELISA kits are used to measure IFNγ secretion. The assay is performed according to manufacturer's directions, and supernatants are tested in duplicate. IFN-γ standards of 1000 pg/ml, 400 pg/ml, 160 pg/ml, 64 pg/ml, 25.6 pg/ml, and 0 pg/ml are tested in duplicate. The assay plate is read on a Molecular Devices Kinetic Microplate Reader and the results analyzed with Vmax Plate Reader/SOFTMAX^{™} software.

T2A2 cells are suitable targets when testing the HPV 16 and 18 E6 and E7 epitopes in the T cell assay (the HPV 2.4-C peptide (SEQ ID NO:61) is included as an example of an HLA-A2 binding HPV epitope to be tested in this way). However, when testing other allotypes (e.g., HLA-A1, -A3, -A11 and BA24), T2A2 cells are not appropriate APCs, and the FluM1 epitope is not an appropriate positive control epitope. Appropriate targets are autologous EBV-transformed cells or PBL. Appropriate control recall epitopes for these other allotypes include the following: HLA-A1, Flu NP 44-52; HLA-A3, Flu NP 265-273; HLA-A11, EBNA3 603-611 and EBNA4 416-424; HLA-A24, EBV LMP 419-427. Suitable *in vitro* immunization controls include HLA-A1, MAGE-1 61-69; HLA-A2, HBVpol 455-463; HLA-A3, *P*. *falciparum* LSA-1 94-102; HLA-A11, HIV gag 325-333; and HLA-A24, HIV gp41 584-591.

### Example 3. Generation of primary peptide-specific cytotoxic T-lymphocytes in vitro using peptide-pulsed autologous dendritic cells

The effectiveness of various HPV-derived peptides in generating CTL responses *in vitro* was determined by co-culturing peptide-pulsed dendritic cells (DC) with peripheral blood mononuclear cells (PBMC) from donors having defined HLA allotypes. The tested peptides included peptides having amino acids sequences corresponding to the following HPV protein amino acid sequences: HPV strain 16 E7 89-97 (SEQ ID NO:96); HPV strain 16 E6 92-101 (SEQ ID NO:77); HPV strain 18 E7 59-67 (SEQ ID NO: 122); HPV strain 18 E6 13-21 (SEQ ID NO:124), and HPV strain 18 E6 97-106 (SEQ ID NO:125).

Primary T cell responses were initiated using a modification of published protocols (Tsai et al., Crit. Rev. Immunol. 18:65-75, 1998; Wilson et al., J. Immunol. 162:3070-78, 1999). Peripheral blood mononuclear cells (PBMC) were purified from leukophoreis products from HLA-A-defined normal donors who screened as seronegative for human immunodeficiency virus and hepatitis B virus. Dendritic cells (DC) were pulsed for 2 h at 20°C with 20 µg/ml peptides in phosphate buffered saline supplemented with 3 µg/ml β2 microglobulin and were irradiated (3000 rad) before use. 2x10⁵ peptide-pulsed and washed DC were co-cultured with 2x10⁶ autologous non-adherent PBMC in 24-well culture plates in the presence of 10 ng/ml IL-7. One day later, cultures were supplemented with 10 ng/ml IL-10. On days 7 and 14, individual cultures were restimulated by transfer of non-adherent PBMC responder cells onto autologous irradiated monocytes pulsed with 20 µg/ml peptide as described above. 10 ng/ml IL-10 and 100 U/ml IL-2 were added 1 and 2 days, respectively, after each restimulation cycle.

The immune reactivity of the PBMC cultures was assessed by the ability to elicit specific IFN-γ secretion from day 21 peptide-sensitized cultures following their incubation with antigen presenting cells pulsed with either the immunizing peptide or an irrelevant antigenic peptide that specifically bound the same targeted HLA class I molecule. 10⁵ effector cells were incubated with 10⁵ autologous, irradiated peptide-pulsed PBMC for 24 h at 37°C in 200 µl complete medium. Supernatants from these cultures were measured for IFN-γ secretion using a commercial ELISA assay. The results are shown in Table 5. Data are presented as picograms of IFN-γ/ml. Specific reactivity by a PBMC culture was arbitrarily defined as at least 20 pg/ml per assay culture and a 1.5-fold or higher difference in IFN-γ secretion in response to the test peptide-pulsed versus the irrelevant peptide-pulsed stimulator cells. FluM1 58-66 and the EBNA4 416-424 CTL peptide epitopes were used as A2 and A11 irrelevant controls, respectively.

**Table 5: In vitro induction of primary, peptide-specific cytotoxic T-lymphocytes using peptide-pulsed dendritic cells**

| | | IFN-γ release (pg/ml) | |
|---|---|---|---|
| | In vitro sensitization/ | Stimulator cells pulsed with | |
| PBMC donor / HLA type | expansion with peptide | irrelevant peptide | test peptide |
| P5 / HLA-A11, A31 | A11 / HIV gag 325-333 | 634 | 1166 |
| P8 / HLA-A2, A28 | A2 / HBV pol 455-463 | 96 | 1824 |
| Specific reactivity against HPV 16 and 18 E6 and E7 peptides by CTL induced with peptide-pulsed dendritic cells. | | | |

| | | IFN-γ release (pg/ml) | |
|---|---|---|---|
| | In vitro sensitization/ | Stimulator cells pulsed with | |
| PBMC donor / HLA type | expansion with peptide | irrelevant peptide | test peptide |
| P5 / HLA-A11, A31 | 16E7 89-97 | 41 | 1275 |
| | 16E6 92-101 | 38 | 259 |
| | 18E7 59-67 | 262 | 665 |
| | | | |
| P8 / HLA-A2, A28 | 18E6 13-21 | 61 | 1590 |
| | 18E6 97-106 | 77 | 1352 |

### Example 4. Generation of a nucleic acid encoding a polyepitope polypeptide

Once candidate epitopes are identified, a DNA fragment is generated that encodes the epitopes of interest. Sequences encoding the epitopes are generated by overlapping PCR or by ligating oligonucleotides. Regulatory elements including a promoter, a Kozak sequence, an initiating ATG, a stop codon, an intron and polyadenylation sequences are included in the construct.

A T7 polymerase promoter is also present at the 5' end of the coding sequence, and a sequence encoding a FLAG mAb (or other) determinant can be present either directly 5' of the first epitope coding sequence or 3' of the last epitope coding sequence. Constructs with and without a targeting signal (e.g., a leader peptide or endosomal/class II loading vesicle targeting sequence) are created. The -3, -1 rules of von Heijne (Nuc. Acids Res. 14:4683-4690, 1986) are followed to ensure the probability of successful cleavage by signal peptidase when the leader is present.

The PCR fragment is cloned into a TA vector (Invitiogen) which permits direct and rapid cloning of PCR products. The construct is sequenced and, if mistakes have been incorporated, the PCR is repeated under more stringent or otherwise optimized conditions. The PCR clone, or alternatively the DNA produced by ligation of oligonucleotides, is then cloned into a mammalian expression vector such as p3K or pcDNA (Invitrogen). In addition, the fragment is cloned into a vaccinia vector (such as pSC11) for generation of recombinant vaccinia virus (see below) and a bacterial expression vector (such as pGEX-5x (Pharmacia)) that permits expression of a GST fusion protein of the polyepitope polypeptide yeast or baculovirus expression vector. *In vitro* transcription/translation (Promega T7 TNT coupled transcription/translation kits) is performed according to the manufacturer's instructions. The translated protein, when analyzed by SDS-PAGE and autoradiography, has been shown to produce a polypeptide of the expected size.

### Example 5. Identification of processed epitopes from polyepitope proteins

The T cell stimulation assay described in Example 2 is used to determine which epitopes are processed from the polyepitope polypeptide encoded by the construct. Effectors generated from PBL stimulated *in vitro* with each of the epitopes encoded in the construct are tested for γIFN secretion when incubated with the appropriate targets. Targets can be autologous PBL cells or EBV-transformed cells infected with a recombinant vaccinia virus that encodes the polyepitope polypeptide (with and without the leader). Processing of the polyepitope polypeptide liberates the T cell epitopes, which are subsequently presented on the surface of the target cells.

Recombinant vaccinia was produced by standard procedures by inserting the polyepitope polypeptide-encoding construct into the SmaI site of the pSC11 vector (Chakrabarti et al., Mol. Cell. Biol. 5:3403-09, 1985) and then generating recombinant vaccinia by methods known in the art.

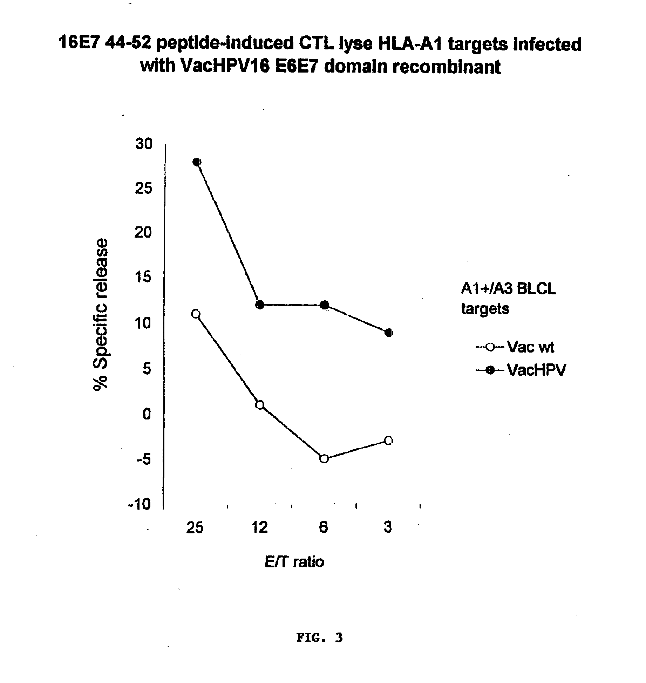

To determine if the encoded polyepitope polypeptide is processed and the expected epitopes are presented by HLA molecules, the *in vitro* stimulation assay may be performed as described in Example 2, with the exception that the targets are autologous EBV-transformed APCs infected with the recombinant vaccinia virus that contains the polyepitope coding sequences. In the experiment illustrated in Fig. 3, EBV-transformed APCs from a donor were infected with recombinant vaccinia virus encoding the test peptide 16E7 44-52, at a m.o.i. of 5 pfu. Control cells were infected with wildtype vaccinia virus. The cells were incubated with the virus for 2.5 h in the presence of ⁵¹Cr. Following washing, the vaccinia-infected target cells were contacted for 5 h with autologous T cells previously activated with 16E7 44-52. ⁵¹Cr release into supernatant was taken as a measure of target cell lysis by the effector cells. As shown in Fig. 3, target cells infected with a vaccinia vector encoding the test peptide were more efficiently lysed by the effector cells than were the control target cells; suggesting that the peptide was expressed within the cells and effectively presented by the cells' HLA molecules.

Targets can instead be autologous PBL infected with the recombinant virus, or 7221 cells expressing a single HLA and which are transfected with the p3k or pcDNA construct (or any other mammalian expression vector) encoding the polyepitope polypeptide.

HLA-A2 transgenic animals may be used to verify that the construct functions to generate HLA-A2 epitopes when delivered *in vivo.* The polyepitope polypeptide-encoding construct in the mammalian expression vector is encapsulated in microparticles. They are injected into HLA-A2 transgenic mice, and T cell responses are subsequently examined as previously described (Hedley et al., Nature Med. 4:365-68, 1998). Alternatively, the expression vector can be delivered as naked DNA. Target cells are HLA-2 expressing cells (e.g., T2A2 or EL4-A2 cells) pulsed with the HLA-A2 binding-epitope being tested, and similar cells infected with the polyepitope-encoding vaccinia vector. In this way, HLA-A2 epitopes that are processed and presented *in vivo* following administration of the construct are identified. Positive results indicate that processing of the polyepitope polypeptide is occurring as predicted, though do not distinguish between epitopes presented by the mouse's endogenous murine MHC molecules and those presented by the transgene-derived HLA-A2 molecules. Immunization of the transgenic mice with a plasmid encoding a polyepitope polypeptide containing a number of HPV-derived epitopes, some of which are known to bind HLA-A2 and at least one of which is known to bind to an endogenous murine MHC molecule, was shown to produce an immune response against two epitopes, including the one known to bind to the murine MHC molecule.

### Example 6: CTL responses generated in DNA-immunized HLA-A2 transgenic mice

CTL responses were measured in mice immunized with microsphere-encapsulated DNA encoding a polyepitope polypeptide. The DNA used for immunization was designated HPV Dra 16/18 (nucleotide sequence of SEQ ID NO:162, encoding the polypeptide of SEQ ID NO:160). Effector cells generated in the DNA immunized mice were tested *in vitro* for their responsiveness in the presence of target cells infected with a vaccinia virus encoding a polyepitope polypeptide. Two polyepitope constructs were separately evaluated in target cells: (1) HPV Dra 16/18 (amino acid sequence of SEQ ID NO:160; nucleotide sequence of SEQ ID NO:162); and (2) HPV 16/18(amino acid sequence of SEQ ID NO:158; nucleotide sequence of SEQ ID NO:161).

Transgenic HLA-A *0201/H-2K^{b} line 6 mice (C57BL/6 x B10.D2) originated from the breeding colony at the Research Institute of Scripps Clinic and were maintained under clean conventional conditions. HLA-A *0201/H-2K^{b} transgenic mice were injected intramuscularly in the hind limbs with 30 µg PEG-DSPE microsphere encapsulated p3KHPVDra 16/18 DNA. The mice were boosted on days 30 and 48 with 50 µg PEG-DSPE encapsulated p3KHPVDra 16/18 DNA.

On day 64, the mice were sacrificed and their spleens harvested. A single cell suspension was prepared, cells pooled, red blood cells lysed, and CD3+ enriched cell population obtained using an immuno-affinity column (R&D Systems, Minneapolis, MN). Spleens cells were restimulated *in vitro* with three day old syngeneic irradiated lipopolysaccharide (LPS)-stimulated B cell blasts (ratio 1:1) which had been preincubated for two hours at 37°C with 100 µM peptide in RPMI 1610 (JRH Biosciences, Lenexa, KS). Peptides used for *in vitro* expansion were: (1) HPV16E648-56 (peptide 124; EVYDFAFRD; SEQ ID NO:163); and (2) HPV16E786-93 (peptide 272; TLGIVCPI; SEQ ID NO:61). Peptides were dissolved in 100% DMSO to 20 mg/ml and stored at -20°C until use. In each well of a 6-well plate 10⁵ effectors were plated in RPMI 1610 supplemented with 10% fetal bovine serum (JRH Biosciences, Lenexa, KS), antibiotics (50 IU/ml penicillin and 50 µg/ml streptomycin), HEPES (JRH Biosciences, Lenexa, KS), 30 µM 2-ME (Gibco-BRL, Gaithersburg, MD) with final peptide concentration of 10 µM. On day 65, 10 IU/ml mIL-2 was added. Effectors were harvested on day 71 and assayed for peptide specific responses by measuring IFN-γ release in an ELISPOT assay, as described below.

The target cell line used in these experiments, an EL4 HLA-A2/H-2K^{b} cell line, was generated by transfecting EL4 cells (C57BL/6 thymoma, H-2^{b}) with a pSV2 plasmid containing the chimeric construct HLA-A2.1 (α₁ and α₂ domain)/H-2K^{b} (α₃ domain) and cotransfecting with the pSV2 neo plasmid containing the neomycin resistance gene.

Two recombinant vaccinia vectors (rVac), Vac HPV Dra 16/18 and Vac HPV 16/18, were generated by the insertion of either pSC11HPV Dra 16/18 or pSC11HPV 16/18 constructs into the thymidine kinase gene of wild type vaccinia virus (a TC-adapted Western Reserve strain; ATCC #VR1354). The resulting recombinant virus underwent three cycles of screening using dual selection with BrdU (inactivation of thymidine kinase activity) and X-gal (pSC11LacZ activity) in host cells with a TK-phenotype (143B, ATCC CRL-8303). Infected targets (EL4 HLA-A2/H-2K^{b} cells) were generated with 10 MOI rVac at 37°C for six hours.

A commercially prepared murine IFN-γ ELISPOT kit (R&D Systems, Minneapolis, MN) was utilized as per the manufacturer's suggested protocol. Each well of a 96-well hydrophobic polyvinylidene flouride (PVDF) membrane backed plate was pre-absorbed with anti-IFN-γ monoclonal antibody (mAb) and blocked with 10% RPMI for 20 minutes. Approximately 10⁴-10⁵ effectors were then mixed with 10⁵ targets (vaccinia infected EL4 HLA-A2/H-2K^{b} cells) for 18-20 hours at 37°C in 5% CO₂. Next, each well was washed four times and incubated overnight at 4°C with a biotinylated non-competing anti-IFN-γ mAb. Wells were then washed three times, incubated for two hours at room temperature with streptavidin alkaline-phosphatase, washed again three times and developed with a 30 minute incubation with BCIP/NBT and washed extensively with distilled water. IFN-γ secreting cells (spots) were enumerated on an automated ELISPOT reader system (Carl Zeiss Inc., Thornwood, NY) with KS ELISPOT Software 4.2 by Zellnet Consulting, Inc. (New York, NY).

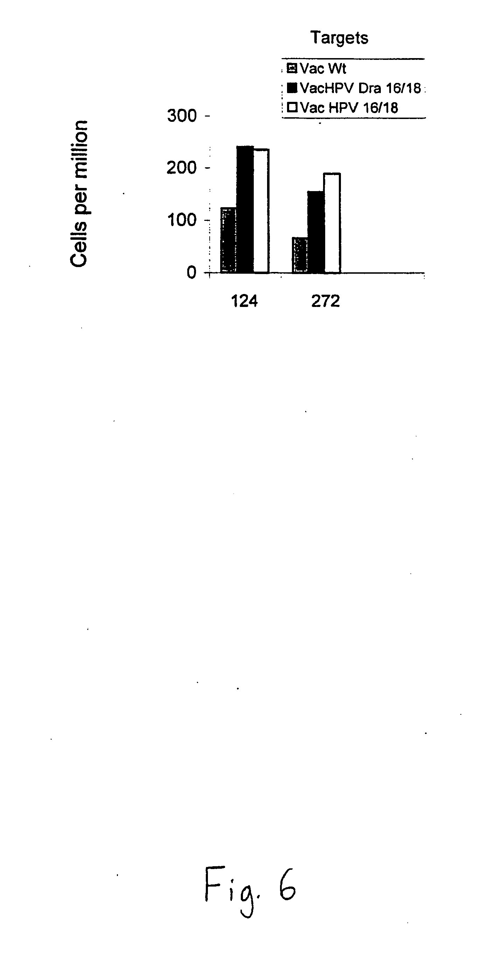

As shown if Figure 6, immunization of mice with DNA encoding an HPV polyepitope construct elicited peptide specific CTLs. Data are presented as the measurement of IFN-γ⁺ spots and represent the antigen specific response per million CD3⁺ cells.

### Example 7: CTL responses in fresh spleen cells from mice treated with DNA encoding a polyepitope polypeptide

Transgenic HLA-A *0201/H-2K^{b} mice (described in Example 6) were sequentially subjected to: (1) an injection of microsphere-encapsulated DNA encoding a polyepitope polypeptide; and (2) an infection with vaccinia virus encoding the polyepitope polypeptide. The IFN-γ ELISPOT assay described in Example 6 was used to detect and enumerate T cells specific for DNA-encoded CTL epitopes in fresh, unexpanded spleen cells.

The treatment regimen was as follows (see Table 6). Ten week old mice were injected with PEG/DSPE microspheres containing 100ug of DNA. Twenty-six days after the microsphere injection, mice were infected intraperitoneally with 1x10⁷ plaque forming units of vaccinia virus encoding the same polyepitope polypeptide.

**Table 6: Vaccination Schedule and Immunization Regimen**

| Experimental Group | Number of Mice | Intramuscular DNA/microsphere injection | Vaccinia Boost | Intraperitoneal Virus dose |
|---|---|---|---|---|
| Untreated | 5 | none | none | none |
| Group 1 | 5 | pBKCMV | Vac wild type | 1x10⁷ PFU/0.1ml |
| Group 2 | 5 | p3KDRaHPV1618 | VacDRα16-18 | 1x10⁷ PFU/0.1ml |

Nine days after the vaccinia boost, spleens were harvested, CD3+ T-cells were enriched (T-cell enrichment columns; R&D Systems, Minneapolis, MN), and peptide-specific IFN-γ release was detected using murine IFN-γ ELISPOT (R&D Systems, Minneapolis, MN). For each antigenic treatment, five wells of 2.5x10⁵ T-enriched spleen cells were co-incubated with 2x10⁵ EL4-A2/K^{b} stimulator cells that were either untreated or pre-pulsed with defined class I peptide epitopes. As controls, spleen cells were also incubated with stimulators either: (1) infected with 20moi of vaccinia wild type virus; or (2) treated with 25 ug Con A/ml. Plates were incubated at 37°C in 10% CO₂ for 48 hours and then developed for IFN-γ detection. HPV-specific IFN-γ responses were reported as the number of spot-forming cells (SFC)/1x10⁶ input T-enriched splenocytes. (the absolute numbers of SFCs are shown in Table 7). The background rate of IFN-γ secretion was defined as SFC/1x10⁶ input T-enriched splenocytes incubated with stimulator cells pulsed with irrelevant peptide (HLA-A2-restricted Plasmodium falciparum cp36 epitope; lot #322). HPV peptide-specific responses were considered positive if twice the background. The frequency of cp36-specific SFC /1x10⁶ cells was 0, 0, and 12 for Groups 0, 1, and 2, respectively.

**Table 7: IFN-γ Responses in Freshly Isolated Spleen Cells**

| Prime | Boost | HPV16 E648-56 | HPV16 E679-87 | HPV16 E749-57 | HPV16 E624-32 | HPV16 E77-15 |
|---|---|---|---|---|---|---|
| p3KDRa HPV1618 | VacHPVDrα 1618 | 124 | 37 | 73 | 24 | 34 |
| Vector | Vac wildtype | 14 | 10 | 2 | 5 | 16 |
| Untreated | none | 6 | 6 | 2 | 0 | 10 |

### Other Embodiments

While the invention has been described in conjunction with the detailed description thereof, the foregoing description is intended to illustrate and not limit the scope of the invention, which is defined by the scope of the appended claims. Other aspects, advantages, and modifications are within the scope of the following claims.

## Claims

1. A nucleic acid encoding a hybrid polypeptide the sequence of which comprises a signal sequence and
(i) at least one of the following segments of human papilloma virus (HPV) strain 16 E6:
AMFQDPQERPRKLPQLCTEL (SEQ ID NO:64),
LLRREVYDFAFRDLCIVYRDGNPY (SEQ ID NO:65), or
KISEYRHYCYSLYGTTLEQQYNK (SEQ ID NO:66);
(ii) at least one of the following segments of HPV strain 16 E7:
TLHEYMLDLQPETTDLYSY (SEQ ID NO:67),
QAEPDRAHYNIVTF (SEQ ID NO:68), or
LLMGTLGIVCPICSQKP (SEQ ID NO:69);
(iii) at least one of the following segments of HPV strain 18 E6:
RRPYKLPDLCTELNTSLQDIEITCVYCKTVLELTEVFEFAFK (SEQ ID NO: 152), or
SVYGDTLEKLTNTGLYNLLIRCLRCQK (SEQ ID NO:153),
(iv) and at least one of the following segments of HPV strain 18 E7:
KATLQDIVLHLEPQNEIPV (SEQ ID NO:154),
HTMLCMCCKCEARI (SEQ ID NO: 155), or
AFQQLFLNTLSFVCPWC (SEQ ID NO:156), provided that the hybrid polypeptide does not comprise a sequence identical to the sequence of either full length, intact E6 or full length, intact E7 protein from HPV strain 16 or 18.

2. The nucleic acid of claim 1, wherein the hybrid polypeptide comprises at least five of the segments.

3. The nucleic acid of claims 1 or 2, wherein the signal sequence is the HLA-DRα leader sequence (SEQ ID NO:63).

4. A plasmid or viral vector comprising the nucleic acid of any one of claims 1 to 3.

5. A microsphere comprising a polymeric matrix or shell and the nucleic acid of any one of claims 1 to 3 or the plasmid or viral vector of claim 4.

6. The microsphere of claim 5, wherein said polymeric matrix or shell consists essentially of a polymer of poly-*co*-glycolic acid (PLGA).

7. A therapeutic composition comprising the nucleic acid of any one of claims 1 to 3 or the plasmid or viral vector of claim 4 and a pharmaceutically acceptable carrier.

8. The therapeutic composition of claim 7, further including an adjuvant.

9. A liposome comprising the nucleic acid of any one of claims 1 to 3 or the plasmid or viral vector of claim 4.

10. The hybrid polypeptide encoded by the nucleic acid of any one of claims 1 to 3.

11. The nucleic acid of any one of claims 1 to 3, the plasmid or viral vector of claim 4, the microsphere of claim 5 or 6, the therapeutic composition of claim 7 or 8, or the liposome of claim 9 for use as a medicament.

12. The nucleic acid of any one of claims 1 to 3, the plasmid or viral vector of claim 4, the microsphere of claim 5 or 6, the therapeutic composition of claim 7 or 8, or the liposome of claim 9 for use in eliciting an immune response in a mammal.

13. The nucleic acid of any one of claims 1 to 3, the plasmid or viral vector of claim 4, the microsphere of claim 5 or 6, the therapeutic composition of claim 7 or 8, or the liposome of claim 9 for use in treating or preventing exophytic condyloma, flat condyloma, cervical cancer, respiratory papilloma, conjunctival papilloma, genital-tract HPV infection, cervical dysplasia, high grade squamous intraepithelial lesions, or anal HPV infection in a mammal.

14. Use of the nucleic acid of any one of claims 1 to 3, the plasmid or viral vector of claim 4, the microsphere of claim 5 or 6, the therapeutic composition of claim 7 or 8, or the liposome of claim 9 in the manufacture of a medicament for use in a) eliciting an immune response in a mammal or b) treating or preventing exophytic condyloma, flat condyloma, cervical cancer, respiratory papilloma, conjunctival papilloma, genital-tract HPV infection, cervical dysplasia, high grade squamous intraepithelial lesions, or anal HPV infection in a mammal.

15. The nucleic acid, plasmid or viral vector, microsphere, therapeutic composition or liposome for use according to claims 12 or 13, or use of claim 14, wherein said nucleic acid, plasmid or viral vector, microsphere, therapeutic composition, liposome or medicament is formulated for administration directly to mucosal tissue.

16. The nucleic acid, plasmid or viral vector, microsphere, therapeutic composition or liposome for use according to claim 15, or use of claim 15, wherein the mucosal tissue is vaginal or anal tissue.

17. The nucleic acid, plasmid or viral vector, microsphere, therapeutic composition or liposome for use according to claims 12 or 13, or use of claim 14, wherein said nucleic acid, plasmid or viral vector, microsphere, therapeutic composition, liposome or medicament is formulated for subcutaneous or intramuscular administration.

18. The nucleic acid, plasmid or viral vector, microsphere, therapeutic composition or liposome for use according to any one of claims 12 to 17, or use of any one of claims 14, 15 or 17, wherein the mammal is human.
